# EUROPEAN PATENT SPECIFICATION

(11) **EP 2 477 652 B1**
(45) Date of publication and mention of the grant of the patent: **15.04.2015**
(21) Application number: 10817780.9
(22) Date of filing: 15.09.2010
(51) Int. Cl.: A61K 39/235, A61K 39/145, C12N 15/00

(54) **IMMUNIZATION STRATEGY TO PREVENT H1N1 INFECTION**
IMMUNISIERUNGSSTRATEGIE ZUR VERHINDERUNG VON H1N1-INFEKTIONEN
STRATÉGIE D'IMMUNISATION POUR EMPÊCHER UNE INFECTION PAR LE H1N1

(30) Priority: 16.09.2009 US 243070 P
(43) Date of publication of application: 25.07.2012
(73) Proprietor: Vaxart, Inc., South San Francisco, CA 94080 (US)
(72) Inventor: TUCKER, Sean, N., San Francisco California 94131 (US); SCALLAN, Ciaran, San Francisco California 94103 (US)
(74) Representative: Campbell, Patrick John Henry
(86) International application number: PCT/US2010/048967
(87) International publication number: WO 2011/034950

(56) References cited:
- WO-A2-2007/100908
- US-A1- 2006 247 421
- US-A1- 2007 269 410
- MATASSOV DEMETRIUS ET AL: "A novel intranasal virus-like particle (VLP) vaccine designed to protect against the pandemic 1918 influenza a virus (H1N1)", VIRAL IMMUNOLOGY, vol. 20, no. 3, September 2007 (2007-09), pages 441-452, XP002687440, ISSN: 0882-8245
- KURT A ZUELKE: "Novel 2009 A/H1N1 Outbreak: USDA ARS Animal Experiment Update", EC BRAINSTORMING MEETING ON THE NOVEL A/H1N1 INFLUENZA AT THE ANIMAL HUMAN INTERFACE, , 6 June 2009 (2009-06-06), pages 1-25, XP008154721, Retrieved from the Internet: URL:http://ec.europa.eu/food/animal/diseas es/influenzaAH1N1/docs/USDA_US_pig_studies .pdf
- ZUELKE: 'Novel 2009 A/H1N1 Outbreak: USDA ARS Animal Experiment Update' EC BRAINSTORMING MEETING ON THE NOVEL A/H1N1 INFLUENZA AT THE ANIMAL HUMAN INTERFACE, [Online] 06 June 2009, XP008154721 Retrieved from the Internet: <URL:http://ec.europa.eu/food/animal/diseas es/influenzaAH1N1/docs/USDA_US_pig_studies. pdf> [retrieved on 2011-02-10]

### SEQUENCE LISTING

<110> Tucker, Sean N. Scallan, Ciaran Vaxart, Inc.
<120> Immunization Strategy to Prevent H1N1
   Infection
<130> 026163-001310PC
<140> WO Not yet assigned
   <141> Not yet assigned
<150> US 61/243,070
   <151> 2009-09-16
<160> 7
<170> FastSEQ for Windows Version 4.0
<210> 1
   <211> 46
   <212> DNA
   <213> Artificial Sequence
<220>
   <223> synthetic dsRNA encoding sequence
<400> 1
   gaaacgatat gggctgaata cttaagtatt cagcccatat cgtttc 46
<210> 2
   <211> 46
   <212> DNA
   <213> Artificial Sequence
<220>
   <223> synthetic dsRNA encoding sequence
<400> 2
   gatggtgctt caagctagta cttaagtact agcttgaagc accatc 46
<210> 3
   <211> 48
   <212> DNA
   <213> Artificial Sequence
<220>
   <223> synthetic dsRNA encoding sequence
<400> 3
   gatggtgctt caagctagta cggatccgta ctagcttgaa gcaccatc 48
<210> 4
   <211> 48
   <212> DNA
   <213> Artificial Sequence
<220>
   <223> synthetic dsRNA encoding sequence
<400> 4
   gaaacgatat gggctgaata cggatccgta ttcagcccat atcgtttc 48
<210> 5
   <211> 46
   <212> DNA
   <213> Artificial Sequence
<220>
   <223> synthetic dsRNA encoding sequence
<400> 5
   cctaataatt atcaaaatgt ggatccacat tttgataatt attagg 46
<210> 6
   <211> 44
   <212> DNA
   <213> Artificial Sequence
<220>
   <223> synthetic dsRNA encoding sequence
<400> 6
   cctaataatt atcaaaatgt aattacattt tgataattat tagg 44
<210> 7
   <211> 34383
   <212> DNA
   <213> Artificial Sequence
<220>
   <223> synthetic recombinant chimeric E1/E3 deleted adenovirus type 5 vector pAd-DX1
<221> modified_base
   <222> (34373)...(34377)
   <223> n = g, a, c or t
<400> 7
   taaggatccc atcatcaata atatacctta ttttggattg aagccaatat gataatgagg 60
   gggtggagtt tgtgacgtgg cgcggggcgt gggaacgggg cgggtgacgt agtagtgtgg 120
   cggaagtgtg atgttgcaag tgtggcggaa cacatgtaag cgacggatgt ggcaaaagtg 180
   acgtttttgg tgtgcgccgg tgtacacagg aagtgacaat tttcgcgcgg ttttaggcgg 240
   atgttgtagt aaatttgggc gtaaccgagt aagatttggc cattttcgcg ggaaaactga 300
   ataagaggaa gtgaaatctg aataattttg tgttactcat agcgcgtaat actgctagag 360
   atctggcgaa agggggatgt gctgcaaggc gattaagttg ggtaacgcca gggttttccc 420
   agtcacgacg ttgtaaaacg acggccagtg aattgtaata cgactcacta tagggcgaat 480
   tgggtactgg ccacagagct tggcccattg catacgttgt atccatatca taatatgtac 540
   atttatattg gctcatgtcc aacattaccg ccatgttgac attgattatt gactagttat 600
   taatagtaat caattacggg gtcattagtt catagcccat atatggagtt ccgcgttaca 660
   taacttacgg taaatggccc gcctggctga ccgcccaacg acccccgccc attgacgtca 720
   ataatgacgt atgttcccat agtaacgcca atagggactt tccattgacg tcaatgggtg 780
   gagtatttac ggtaaactgc ccacttggca gtacatcaag tgtatcatat gccaagtacg 840
   ccccctattg acgtcaatga cggtaaatgg cccgcctggc attatgccca gtacatgacc 900
   ttatgggact ttcctacttg gcagtacatc tacgtattag tcatcgctat taccatggtg 960
   atgcggtttt ggcagtacat caatgggcgt ggatagcggt ttgactcacg gggatttcca 1020
   agtctccacc ccattgacgt caatgggagt ttgttttggc accaaaatca acgggacttt 1080
   ccaaaatgtc gtaacaactc cgccccattg acgcaaatgg gcggtaggcg tgtacggtgg 1140
   gaggtctata taagcagagc tcgtttagtg aaccgtcaga tcgcctggag acgccatcca 1200
   cgctgttttg acctccatag aagacaccgg gaccgatcca gcctgactct agcctagctc 1260
   tgaagttggt ggtgaggccc tgggcaggtt ggtatcaagg ttacaagaca ggtttaagga 1320
   gaccaataga aactgggcat gtggagacag agaagactct tgggtttctg ataggcactg 1380
   actctctctg cctattggtc tattttccca cccttaggct gctggtctga gcctaggaga 1440
   tctctcgagg tcgacggtat cgatgggtac cgccaccatg aaggctatac ttgtcgtact 1500
   gctctacacg ttcgctactg ctaacgccga caccctctgc attggatacc atgccaataa 1560
   ctctaccgat actgtggaca cggtgctgga aaaaaacgtg acagtaacac actcagtgaa 1620
   ccttctcgaa gacaagcaca atggcaaact gtgcaagctg cggggcgttg ctcctctgca 1680
   cctgggcaaa tgtaacatag ccggctggat tcttggcaat ccagagtgcg aatctttgag 1740
   caccgcaagc agctggagtt acattgtgga aaccccgtcc tccgataacg gaacttgcta 1800
   cccaggagac tttatcgact acgaggaact gagagagcag ctcagctccg tgtcttcctt 1860
   cgaacgcttt gagatcttcc caaaaacatc ctcctggcca aaccacgata gtaacaaggg 1920
   ggtaacagcc gcgtgcccac atgcaggggc taaatcattt tataagaacc tcatctggct 1980
   cgtgaagaag ggtaacagct acccaaaact ttctaagtct tatataaacg ataagggcaa 2040
   ggaggttctg gtcctgtggg gtatacacca tccctctacc agtgcagacc agcaatcatt 2100
   gtatcagaac gctgacacat acgttttcgt aggctctagc agatattcca agaaattcaa 2160
   gccagagatc gccatcaggc ctaaggtccg cgaccaggag ggccgaatga actattactg 2220
   gacactcgtc gaacctgggg ataagattac ctttgaggcc actggtaatc tggttgttcc 2280
   tagatacgca ttcgctatgg agcgcaatgc cggtagtggc atcataatct ctgacacccc 2340
   cgttcacgac tgtaatacca cttgccagac ccctaaagga gcaatcaata cctcattgcc 2400
   cttccagaat atccatccca tcacaatagg aaagtgccct aaatacgtga aaagcaccaa 2460
   gctgcggttg gccactggtc tgcggaatat accgagcatc cagtctcgag gactcttcgg 2520
   ggcaatcgct gggtttatcg agggcggctg gaccgggatg gtcgacggtt ggtacggata 2580
   ccaccatcag aacgagcaag ggtccggcta tgccgcagac ctcaagtcta cccagaacgc 2640
   catcgatgag attacgaata aagtaaattc cgtgatcgag aagatgaata cacagtttac 2700
   agccgtgggg aaggagttca accatctgga aaagcggatt gaaaacctga ataaaaaagt 2760
   ggatgatgga tttttggata tttggactta caacgccgag ctgctcgttc tgctggagaa 2820
   tgaaagaacc ctggattacc acgatagtaa cgtgaagaac ctttacgaga aagttaggag 2880
   ccaactgaag aataacgcta aagaaatcgg caacggttgc tttgaattct accacaagtg 2940
   cgataacaca tgcatggagt ctgttaaaaa tgggacttac gactatccta aatactccga 3000
   ggaagccaag ttgaatagag aggagatcga tggagtcaag ctcgagagca cgcgcattta 3060
   ccagatactg gccatttatt ccacagttgc gtcatccctg gtgctcgttg tgagcttggg 3120
   ggccatatcc ttctggatgt gctctaacgg gagcctccag tgtcgaatct gcatcggtac 3180
   cggcggccgc cctattctat agtgtcacct aaatgctaga gctcgctgat cagcctcgac 3240
   tgtgccttct agttgccagc catctgttgt ttgcccctcc cccgtgcctt ccttgaccct 3300
   ggaaggtgcc actcccactg tcctttccta ataaaatgag gaaattgcat cgcattgtct 3360
   gagtaggtgt cattctattc tggggggtgg ggtggggcag gacagcaagg gggaggattg 3420
   ggaagacaat agcaggcatg ctggggatgc ggtgggctct atggcttctg aggcggaaag 3480
   aaccaaagct tagtaatcaa ttacggggtc attagttcat agcccatata tggagttccg 3540
   cgttacataa cttacggtaa atggcccgcc tggctgaccg cccaacgacc cccgcccatt 3600
   gacgtcaata atgacgtatg ttcccatagt aacgccaata gggactttcc attgacgtca 3660
   atgggtggag tatttacggt aaactgccca cttggcagta catcaagtgt atcatatgcc 3720
   aagtacgccc cctattgacg tcaatgacgg taaatggccc gcctggcatt atgcccagta 3780
   catgacctta tgggactttc ctacttggca gtacatctac gtattagtca tcgctattac 3840
   catggtgatg cggttttggc agtacatcaa tgggcgtgga tagcggtttg actcacgggg 3900
   atttccaagt ctccacccca ttgacgtcaa tgggagtttg ttttggcacc aaaatcaacg 3960
   ggactttcca aaatgtcgta acaactccgc cccattgacg caaatgggcg gtaggcgtgt 4020
   acggtgggag gtctatataa gcagagctgg tttagtgaac cgtcagatcc gctagagatc 4080
   tgggaaacga tatgggctga atacggatcc gtattcagcc catatcgttt ctctagaaat 4140
   aaaatatctt tattttcatt acatctgtgt gttggttttt tgtgtggcgg ccgctcgagc 4200
   ctaagcttct agataagata tccgatccac cggatctaga taactgatca taatcagcca 4260
   taccacattt gtagaggttt tacttgcttt aaaaaacctc ccacacctcc ccctgaacct 4320
   gaaacataaa atgaatgcaa ttgttgttgt taacttgttt attgcagctt ataatggtta 4380
   caaataaagc aatagcatca caaatttcac aaataaagca tttttttcac tgcattctag 4440
   ttgtggtttg tccaaactca tcaatgtatc ttaacgcgga tctgggcgtg gttaagggtg 4500
   ggaaagaata tataaggtgg gggtcttatg tagttttgta tctgttttgc agcagccgcc 4560
   gccgccatga gcaccaactc gtttgatgga agcattgtga gcttgtcgac tcgaagaatc 4620
   tgggcgtggt taagggtggg aaagaatata taaggtgggg gtcttatgta gttttgtatc 4680
   tgttttgcag cagccgccgc cgccatgagc accaactcgt ttgatggaag cattgtgagc 4740
   tcatatttga caacgcgcat gcccccatgg gccggggtgc gtcagaatgt gatgggctcc 4800
   agcattgatg gtcgccccgt cctgcccgca aactctacta ccttgaccta cgagaccgtg 4860
   tctggaacgc cgttggagac tgcagcctcc gccgccgctt cagccgctgc agccaccgcc 4920
   cgcgggattg tgactgactt tgctttcctg agcccgcttg caagcagtgc agcttcccgt 4980
   tcatccgccc gcgatgacaa gttgacggct cttttggcac aattggattc tttgacccgg 5040
   gaacttaatg tcgtttctca gcagctgttg gatctgcgcc agcaggtttc tgccctgaag 5100
   gcttcctccc ctcccaatgc ggtttaaaac ataaataaaa aaccagactc tgtttggatt 5160
   tggatcaagc aagtgtcttg ctgtctttat ttaggggttt tgcgcgcgcg gtaggcccgg 5220
   gaccagcggt ctcggtcgtt gagggtcctg tgtatttttt ccaggacgtg gtaaaggtga 5280
   ctctggatgt tcagatacat gggcataagc ccgtctctgg ggtggaggta gcaccactgc 5340
   agagcttcat gctgcggggt ggtgttgtag atgatccagt cgtagcagga gcgctgggcg 5400
   tggtgcctaa aaatgtcttt cagtagcaag ctgattgcca ggggcaggcc cttggtgtaa 5460
   gtgtttacaa agcggttaag ctgggatggg tgcatacgtg gggatatgag atgcatcttg 5520
   gactgtattt ttaggttggc tatgttccca gccatatccc tccggggatt catgttgtgc 5580
   agaaccacca gcacagtgta tccggtgcac ttgggaaatt tgtcatgtag cttagaagga 5640
   aatgcgtgga agaacttgga gacgcccttg tgacctccaa gattttccat gcattcgtcc 5700
   ataatgatgg caatgggccc acgggcggcg gcctgggcga agatatttct gggatcacta 5760
   acgtcatagt tgtgttccag gatgagatcg tcataggcca tttttacaaa gcgcgggcgg 5820
   agggtgccag actgcggtat aatggttcca tccggcccag gggcgtagtt accctcacag 5880
   atttgcattt cccacgcttt gagttcagat ggggggatca tgtctacctg cggggcgatg 5940
   aagaaaacgg tttccggggt aggggagatc agctgggaag aaagcaggtt cctgagcagc 6000
   tgcgacttac cgcagccggt gggcccgtaa atcacaccta ttaccgggtg caactggtag 6060
   ttaagagagc tgcagctgcc gtcatccctg agcagggggg ccacttcgtt aagcatgtcc 6120
   ctgactcgca tgttttccct gaccaaatcc gccagaaggc gctcgccgcc cagcgatagc 6180
   agttcttgca aggaagcaaa gtttttcaac ggtttgagac cgtccgccgt aggcatgctt 6240
   ttgagcgttt gaccaagcag ttccaggcgg tcccacagct cggtcacctg ctctacggca 6300
   tctcgatcca gcatatctcc tcgtttcgcg ggttggggcg gctttcgctg tacggcagta 6360
   gtcggtgctc gtccagacgg gccagggtca tgtctttcca cgggcgcagg gtcctcgtca 6420
   gcgtagtctg ggtcacggtg aaggggtgcg ctccgggctg cgcgctggcc agggtgcgct 6480
   tgaggctggt cctgctggtg ctgaagcgct gccggtcttc gccctgcgcg tcggccaggt 6540
   agcatttgac catggtgtca tagtccagcc cctccgcggc gtggcccttg gcgcgcagct 6600
   tgcccttgga ggaggcgccg cacgaggggc agtgcagact tttgagggcg tagagcttgg 6660
   gcgcgagaaa taccgattcc ggggagtagg catccgcgcc gcaggccccg cagacggtct 6720
   cgcattccac gagccaggtg agctctggcc gttcggggtc aaaaaccagg tttcccccat 6780
   gctttttgat gcgtttctta cctctggttt ccatgagccg gtgtccacgc tcggtgacga 6840
   aaaggctgtc cgtgtccccg tatacagact tgagaggcct gtcctcgagc ggtgttccgc 6900
   ggtcctcctc gtatagaaac tcggaccact ctgagacaaa ggctcgcgtc caggccagca 6960
   cgaaggaggc taagtgggag gggtagcggt cgttgtccac tagggggtcc actcgctcca 7020
   gggtgtgaag acacatgtcg ccctcttcgg catcaaggaa ggtgattggt ttgtaggtgt 7080
   aggccacgtg accgggtgtt cctgaagggg ggctataaaa gggggtgggg gcgcgttcgt 7140
   cctcactctc ttccgcatcg ctgtctgcga gggccagctg ttggggtgag tactccctct 7200
   gaaaagcggg catgacttct gcgctaagat tgtcagtttc caaaaacgag gaggatttga 7260
   tattcacctg gcccgcggtg atgcctttga gggtggccgc atccatctgg tcagaaaaga 7320
   caatcttttt gttgtcaagc ttggtggcaa acgacccgta gagggcgttg gacagcaact 7380
   tggcgatgga gcgcagggtt tggtttttgt cgcgatcggc gcgctccttg gccgcgatgt 7440
   ttagctgcac gtattcgcgc gcaacgcacc gccattcggg aaagacggtg gtgcgctcgt 7500
   cgggcaccag gtgcacgcgc caaccgcggt tgtgcagggt gacaaggtca acgctggtgg 7560
   ctacctctcc gcgtaggcgc tcgttggtcc agcagaggcg gccgcccttg cgcgagcaga 7620
   atggcggtag ggggtctagc tgcgtctcgt ccggggggtc tgcgtccacg gtaaagaccc 7680
   cgggcagcag gcgcgcgtcg aagtagtcta tcttgcatcc ttgcaagtct agcgcctgct 7740
   gccatgcgcg ggcggcaagc gcgcgctcgt atgggttgag tgggggaccc catggcatgg 7800
   ggtgggtgag cgcggaggcg tacatgccgc aaatgtcgta aacgtagagg ggctctctga 7860
   gtattccaag atatgtaggg tagcatcttc caccgcggat gctggcgcgc acgtaatcgt 7920
   atagttcgtg cgagggagcg aggaggtcgg gaccgaggtt gctacgggcg ggctgctctg 7980
   ctcggaagac tatctgcctg aagatggcat gtgagttgga tgatatggtt ggacgctgga 8040
   agacgttgaa gctggcgtct gtgagaccta ccgcgtcacg cacgaaggag gcgtaggagt 8100
   cgcgcagctt gttgaccagc tcggcggtga cctgcacgtc tagggcgcag tagtccaggg 8160
   tttccttgat gatgtcatac ttatcctgtc cctttttttt ccacagctcg cggttgagga 8220
   caaactcttc gcggtctttc cagtactctt ggatcggaaa cccgtcggcc tccgaacggt 8280
   aagagcctag catgtagaac tggttgacgg cctggtaggc gcagcatccc ttttctacgg 8340
   gtagcgcgta tgcctgcgcg gccttccgga gcgaggtgtg ggtgagcgca aaggtgtccc 8400
   tgaccatgac tttgaggtac tggtatttga agtcagtgtc gtcgcatccg ccctgctccc 8460
   agagcaaaaa gtccgtgcgc tttttggaac gcggatttgg cagggcgaag gtgacatcgt 8520
   tgaagagtat ctttcccgcg cgaggcataa agttgcgtgt gatgcggaag ggtcccggca 8580
   cctcggaacg gttgttaatt acctgggcgg cgagcacgat ctcgtcaaag ccgttgatgt 8640
   tgtggcccac aatgtaaagt tccaagaagc gcgggatgcc cttgatggaa ggcaattttt 8700
   taagttcctc gtaggtgagc tcttcagggg agctgagccc gtgctctgaa agggcccagt 8760
   ctgcaagatg agggttggaa gcgacgaatg agctccacag gtcacgggcc attagcattt 8820
   gcaggtggtc gcgaaaggtc ctaaactggc gacctatggc cattttttct ggggtgatgc 8880
   agtagaaggt aagcgggtct tgttcccagc ggtcccatcc aaggttcgcg gctaggtctc 8940
   gcgcggcagt cactagaggc tcatctccgc cgaacttcat gaccagcatg aagggcacga 9000
   gctgcttccc aaaggccccc atccaagtat aggtctctac atcgtaggtg acaaagagac 9060
   gctcggtgcg aggatgcgag ccgatcggga agaactggat ctcccgccac caattggagg 9120
   agtggctatt gatgtggtga aagtagaagt ccctgcgacg ggccgaacac tcgtgctggc 9180
   ttttgtaaaa acgtgcgcag tactggcagc ggtgcacggg ctgtacatcc tgcacgaggt 9240
   tgacctgacg accgcgcaca aggaagcaga gtgggaattt gagcccctcg cctggcgggt 9300
   ttggctggtg gtcttctact tcggctgctt gtccttgacc gtctggctgc tcgaggggag 9360
   ttacggtgga tcggaccacc acgccgcgcg agcccaaagt ccagatgtcc gcgcgcggcg 9420
   gtcggagctt gatgacaaca tcgcgcagat gggagctgtc catggtctgg agctcccgcg 9480
   gcgtcaggtc aggcgggagc tcctgcaggt ttacctcgca tagacgggtc agggcgcggg 9540
   ctagatccag gtgataccta atttccaggg gctggttggt ggcggcgtcg atggcttgca 9600
   agaggccgca tccccgcggc gcgactacgg taccgcgcgg cgggcggtgg gccgcggggg 9660
   tgtccttgga tgatgcatct aaaagcggtg acgcgggcga gcccccggag gtaggggggg 9720
   ctccggaccc gccgggagag gggcaggggg cacgtcggcg ccgcgcgcgg gcaggagctg 9780
   gtgctgcgcg cgtaggttgc tggcgaacgc gacgacgcgg cggttgatct cctgaatctg 9840
   gcgcctctgc gtgaagacga cgggcccggt gagcttgagc ctgaaagaga gttcgacaga 9900
   atcaatttcg gtgtcgttga cggcggcctg gcgcaaaatc tcctgcacgt ctcctgagtt 9960
   gtcttgatag gcgatctcgg ccatgaactg ctcgatctct tcctcctgga gatctccgcg 10020
   tccggctcgc tccacggtgg cggcgaggtc gttggaaatg cgggccatga gctgcgagaa 10080
   ggcgttgagg cctccctcgt tccagacgcg gctgtagacc acgccccctt cggcatcgcg 10140
   ggcgcgcatg accacctgcg cgagattgag ctccacgtgc cgggcgaaga cggcgtagtt 10200
   tcgcaggcgc tgaaagaggt agttgagggt ggtggcggtg tgttctgcca cgaagaagta 10260
   cataacccag cgtcgcaacg tggattcgtt gatatccccc aaggcctcaa ggcgctccat 10320
   ggcctcgtag aagtccacgg cgaagttgaa aaactgggag ttgcgcgccg acacggttaa 10380
   ctcctcctcc agaagacgga tgagctcggc gacagtgtcg cgcacctcgc gctcaaaggc 10440
   tacaggggcc tcttcttctt cttcaatctc ctcttccata agggcctccc cttcttcttc 10500
   ttctggcggc ggtgggggag gggggacacg gcggcgacga cggcgcaccg ggaggcggtc 10560
   gacaaagcgc tcgatcatct ccccgcggcg acggcgcatg gtctcggtga cggcgcggcc 10620
   gttctcgcgg gggcgcagtt ggaagacgcc gcccgtcatg tcccggttat gggttggcgg 10680
   ggggctgcca tgcggcaggg atacggcgct aacgatgcat ctcaacaatt gttgtgtagg 10740
   tactccgccg ccgagggacc tgagcgagtc cgcatcgacc ggatcggaaa acctctcgag 10800
   aaaggcgtct aaccagtcac agtcgcaagg taggctgagc accgtggcgg gcggcagcgg 10860
   gcggcggtcg gggttgtttc tggcggaggt gctgctgatg atgtaattaa agtaggcggt 10920
   cttgagacgg cggatggtcg acagaagcac catgtccttg ggtccggcct gctgaatgcg 10980
   caggcggtcg gccatgcccc aggcttcgtt ttgacatcgg cgcaggtctt tgtagtagtc 11040
   ttgcatgagc ctttctaccg gcacttcttc ttctccttcc tcttgtcctg catctcttgc 11100
   atctatcgct gcggcggcgg cggagtttgg ccgtaggtgg cgccctcttc ctcccatgcg 11160
   tgtgaccccg aagcccctca tcggctgaag cagggctagg tcggcgacaa cgcgctcggc 11220
   taatatggcc tgctgcacct gcgtgagggt agactggaag tcatccatgt ccacaaagcg 11280
   gtggtatgcg cccgtgttga tggtgtaagt gcagttggcc ataacggacc agttaacggt 11340
   ctggtgaccc ggctgcgaga gctcggtgta cctgagacgc gagtaagccc tcgagtcaaa 11400
   tacgtagtcg ttgcaagtcc gcaccaggta ctggtatccc accaaaaagt gcggcggcgg 11460
   ctggcggtag aggggccagc gtagggtggc cggggctccg ggggcgagat cttccaacat 11520
   aaggcgatga tatccgtaga tgtacctgga catccaggtg atgccggcgg cggtggtgga 11580
   ggcgcgcgga aagtcgcgga cgcggttcca gatgttgcgc agcggcaaaa agtgctccat 11640
   ggtcgggacg ctctggccgg tcaggcgcgc gcaatcgttg acgctctacc gtgcaaaagg 11700
   agagcctgta agcgggcact cttccgtggt ctggtggata aattcgcaag ggtatcatgg 11760
   cggacgaccg gggttcgagc cccgtatccg gccgtccgcc gtgatccatg cggttaccgc 11820
   ccgcgtgtcg aacccaggtg tgcgacgtca gacaacgggg gagtgctcct tttggcttcc 11880
   ttccaggcgc ggcggctgct gcgctagctt ttttggccac tggccgcgcg cagcgtaagc 11940
   ggttaggctg gaaagcgaaa gcattaagtg gctcgctccc tgtagccgga gggttatttt 12000
   ccaagggttg agtcgcggga cccccggttc gagtctcgga ccggccggac tgcggcgaac 12060
   gggggtttgc ctccccgtca tgcaagaccc cgcttgcaaa ttcctccgga aacagggacg 12120
   agcccctttt ttgcttttcc cagatgcatc cggtgctgcg gcagatgcgc ccccctcctc 12180
   agcagcggca agagcaagag cagcggcaga catgcagggc accctcccct cctcctaccg 12240
   cgtcaggagg ggcgacatcc gcggttgacg cggcagcaga tggtgattac gaacccccgc 12300
   ggcgccgggc ccggcactac ctggacttgg aggagggcga gggcctggcg cggctaggag 12360
   cgccctctcc tgagcggtac ccaagggtgc agctgaagcg tgatacgcgt gaggcgtacg 12420
   tgccgcggca gaacctgttt cgcgaccgcg agggagagga gcccgaggag atgcgggatc 12480
   gaaagttcca cgcagggcgc gagctgcggc atggcctgaa tcgcgagcgg ttgctgcgcg 12540
   aggaggactt tgagcccgac gcgcgaaccg ggattagtcc cgcgcgcgca cacgtggcgg 12600
   ccgccgacct ggtaaccgca tacgagcaga cggtgaacca ggagattaac tttcaaaaaa 12660
   gctttaacaa ccacgtgcgt acgcttgtgg cgcgcgagga ggtggctata ggactgatgc 12720
   atctgtggga ctttgtaagc gcgctggagc aaaacccaaa tagcaagccg ctcatggcgc 12780
   agctgttcct tatagtgcag cacagcaggg acaacgaggc attcagggat gcgctgctaa 12840
   acatagtaga gcccgagggc cgctggctgc tcgatttgat aaacatcctg cagagcatag 12900
   tggtgcagga gcgcagcttg agcctggctg acaaggtggc cgccatcaac tattccatgc 12960
   ttagcctggg caagttttac gcccgcaaga tataccatac cccttacgtt cccatagaca 13020
   aggaggtaaa gatcgagggg ttctacatgc gcatggcgct gaaggtgctt accttgagcg 13080
   acgacctggg cgtttatcgc aacgagcgca tccacaaggc cgtgagcgtg agccggcggc 13140
   gcgagctcag cgaccgcgag ctgatgcaca gcctgcaaag ggccctggct ggcacgggca 13200
   gcggcgatag agaggccgag tcctactttg acgcgggcgc tgacctgcgc tgggccccaa 13260
   gccgacgcgc cctggaggca gctggggccg gacctgggct ggcggtggca cccgcgcgcg 13320
   ctggcaacgt cggcggcgtg gaggaatatg acgaggacga tgagtacgag ccagaggacg 13380
   gcgagtacta agcggtgatg tttctgatca gatgatgcaa gacgcaacgg acccggcggt 13440
   gcgggcggcg ctgcagagcc agccgtccgg ccttaactcc acggacgact ggcgccaggt 13500
   catggaccgc atcatgtcgc tgactgcgcg caatcctgac gcgttccggc agcagccgca 13560
   ggccaaccgg ctctccgcaa ttctggaagc ggtggtcccg gcgcgcgcaa accccacgca 13620
   cgagaaggtg ctggcgatcg taaacgcgct ggccgaaaac agggccatcc ggcccgacga 13680
   ggccggcctg gtctacgacg cgctgcttca gcgcgtggct cgttacaaca gcggcaacgt 13740
   gcagaccaac ctggaccggc tggtggggga tgtgcgcgag gccgtggcgc agcgtgagcg 13800
   cgcgcagcag cagggcaacc tgggctccat ggttgcacta aacgccttcc tgagtacaca 13860
   gcccgccaac gtgccgcggg gacaggagga ctacaccaac tttgtgagcg cactgcggct 13920
   aatggtgact gagacaccgc aaagtgaggt gtaccagtct gggccagact attttttcca 13980
   gaccagtaga caaggcctgc agaccgtaaa cctgagccag gctttcaaaa acttgcaggg 14040
   gctgtggggg gtgcgggctc ccacaggcga ccgcgcgacc gtgtctagct tgctgacgcc 14100
   caactcgcgc ctgttgctgc tgctaatagc gcccttcacg gacagtggca gcgtgtcccg 14160
   ggacacatac ctaggtcact tgctgacact gtaccgcgag gccataggtc aggcgcatgt 14220
   ggacgagcat actttccagg agattacaag tgtcagccgc gcgctggggc aggaggacac 14280
   gggcagcctg gaggcaaccc taaactacct gctgaccaac cggcggcaga agatcccctc 14340
   gttgcacagt ttaaacagcg aggaggagcg cattttgcgc tacgtgcagc agagcgtgag 14400
   ccttaacctg atgcgcgacg gggtaacgcc cagcgtggcg ctggacatga ccgcgcgcaa 14460
   catggaaccg ggcatgtatg cctcaaaccg gccgtttatc aaccgcctaa tggactactt 14520
   gcatcgcgcg gccgccgtga accccgagta tttcaccaat gccatcttga acccgcactg 14580
   gctaccgccc cctggtttct acaccggggg attcgaggtg cccgagggta acgatggatt 14640
   cctctgggac gacatagacg acagcgtgtt ttccccgcaa ccgcagaccc tgctagagtt 14700
   gcaacagcgc gagcaggcag aggcggcgct gcgaaaggaa agcttccgca ggccaagcag 14760
   cttgtccgat ctaggcgctg cggccccgcg gtcagatgct agtagcccat ttccaagctt 14820
   gatagggtct cttaccagca ctcgcaccac ccgcccgcgc ctgctgggcg aggaggagta 14880
   cctaaacaac tcgctgctgc agccgcagcg cgaaaaaaac ctgcctccgg catttcccaa 14940
   caacgggata gagagcctag tggacaagat gagtagatgg aagacgtacg cgcaggagca 15000
   cagggacgtg ccaggcccgc gcccgcccac ccgtcgtcaa aggcacgacc gtcagcgggg 15060
   tctggtgtgg gaggacgatg actcggcaga cgacagcagc gtcctggatt tgggagggag 15120
   tggcaacccg tttgcgcacc ttcgccccag gctggggaga atgttttaaa aaaaaaaaag 15180
   catgatgcaa aataaaaaac tcaccaaggc catggcaccg agcgttggtt ttcttgtatt 15240
   ccccttagta tgcggcgcgc ggcgatgtat gaggaaggtc ctcctccctc ctacgagagt 15300
   gtggtgagcg cggcgccagt ggcggcggcg ctgggttctc ccttcgatgc tcccctggac 15360
   ccgccgtttg tgcctccgcg gtacctgcgg cctaccgggg ggagaaacag catccgttac 15420
   tctgagttgg cacccctatt cgacaccacc cgtgtgtacc tggtggacaa caagtcaacg 15480
   gatgtggcat ccctgaacta ccagaacgac cacagcaact ttctgaccac ggtcattcaa 15540
   aacaatgact acagcccggg ggaggcaagc acacagacca tcaatcttga cgaccggtcg 15600
   cactggggcg gcgacctgaa aaccatcctg cataccaaca tgccaaatgt gaacgagttc 15660
   atgtttacca ataagtttaa ggcgcgggtg atggtgtcgc gcttgcctac taaggacaat 15720
   caggtggagc tgaaatacga gtgggtggag ttcacgctgc ccgagggcaa ctactccgag 15780
   accatgacca tagaccttat gaacaacgcg atcgtggagc actacttgaa agtgggcaga 15840
   cagaacgggg ttctggaaag cgacatcggg gtaaagtttg acacccgcaa cttcagactg 15900
   gggtttgacc ccgtcactgg tcttgtcatg cctggggtat atacaaacga agccttccat 15960
   ccagacatca ttttgctgcc aggatgcggg gtggacttca cccacagccg cctgagcaac 16020
   ttgttgggca tccgcaagcg gcaacccttc caggagggct ttaggatcac ctacgatgat 16080
   ctggagggtg gtaacattcc cgcactgttg gatgtggacg cctaccaggc gagcttgaaa 16140
   gatgacaccg aacagggcgg gggtggcgca ggcggcagca acagcagtgg cagcggcgcg 16200
   gaagagaact ccaacgcggc agccgcggca atgcagccgg tggaggacat gaacgatcat 16260
   gccattcgcg gcgacacctt tgccacacgg gctgaggaga agcgcgctga ggccgaagca 16320
   gcggccgaag ctgccgcccc cgctgcgcaa cccgaggtcg agaagcctca gaagaaaccg 16380
   gtgatcaaac ccctgacaga ggacagcaag aaacgcagtt acaacctaat aagcaatgac 16440
   agcaccttca cccagtaccg cagctggtac cttgcataca actacggcga ccctcagacc 16500
   ggaatccgct catggaccct gctttgcact cctgacgtaa cctgcggctc ggagcaggtc 16560
   tactggtcgt tgccagacat gatgcaagac cccgtgacct tccgctccac gcgccagatc 16620
   agcaactttc cggtggtggg cgccgagctg ttgcccgtgc actccaagag cttctacaac 16680
   gaccaggccg tctactccca actcatccgc cagtttacct ctctgaccca cgtgttcaat 16740
   cgctttcccg agaaccagat tttggcgcgc ccgccagccc ccaccatcac caccgtcagt 16800
   gaaaacgttc ctgctctcac agatcacggg acgctaccgc tgcgcaacag catcggagga 16860
   gtccagcgag tgaccattac tgacgccaga cgccgcacct gcccctacgt ttacaaggcc 16920
   ctgggcatag tctcgccgcg cgtcctatcg agccgcactt tttgagcaag catgtccatc 16980
   cttatatcgc ccagcaataa cacaggctgg ggcctgcgct tcccaagcaa gatgtttggc 17040
   ggggccaaga agcgctccga ccaacaccca gtgcgcgtgc gcgggcacta ccgcgcgccc 17100
   tggggcgcgc acaaacgcgg ccgcactggg cgcaccaccg tcgatgacgc catcgacgcg 17160
   gtggtggagg aggcgcgcaa ctacacgccc acgccgccac cagtgtccac agtggacgcg 17220
   gccattcaga ccgtggtgcg cggagcccgg cgctatgcta aaatgaagag acggcggagg 17280
   cgcgtagcac gtcgccaccg ccgccgaccc ggcactgccg cccaacgcgc ggcggcggcc 17340
   ctgcttaacc gcgcacgtcg caccggccga cgggcggcca tgcgggccgc tcgaaggctg 17400
   gccgcgggta ttgtcactgt gccccccagg tccaggcgac gagcggccgc cgcagcagcc 17460
   gcggccatta gtgctatgac tcagggtcgc aggggcaacg tgtattgggt gcgcgactcg 17520
   gttagcggcc tgcgcgtgcc cgtgcgcacc cgccccccgc gcaactagat tgcaagaaaa 17580
   aactacttag actcgtactg ttgtatgtat ccagcggcgg cggcgcgcaa cgaagctatg 17640
   tccaagcgca aaatcaaaga agagatgctc caggtcatcg cgccggagat ctatggcccc 17700
   ccgaagaagg aagagcagga ttacaagccc cgaaagctaa agcgggtcaa aaagaaaaag 17760
   aaagatgatg atgatgaact tgacgacgag gtggaactgc tgcacgctac cgcgcccagg 17820
   cgacgggtac agtggaaagg tcgacgcgta aaacgtgttt tgcgacccgg caccaccgta 17880
   gtctttacgc ccggtgagcg ctccacccgc acctacaagc gcgtgtatga tgaggtgtac 17940
   ggcgacgagg acctgcttga gcaggccaac gagcgcctcg gggagtttgc ctacggaaag 18000
   cggcataagg acatgctggc gttgccgctg gacgagggca acccaacacc tagcctaaag 18060
   cccgtaacac tgcagcaggt gctgcccgcg cttgcaccgt ccgaagaaaa gcgcggccta 18120
   aagcgcgagt ctggtgactt ggcacccacc gtgcagctga tggtacccaa gcgccagcga 18180
   ctggaagatg tcttggaaaa aatgaccgtg gaacctgggc tggagcccga ggtccgcgtg 18240
   cggccaatca agcaggtggc gccgggactg ggcgtgcaga ccgtggacgt tcagataccc 18300
   actaccagta gcaccagtat tgccaccgcc acagagggca tggagacaca aacgtccccg 18360
   gttgcctcag cggtggcgga tgccgcggtg caggcggtcg ctgcggccgc gtccaagacc 18420
   tctacggagg tgcaaacgga cccgtggatg tttcgcgttt cagccccccg gcgcccgcgc 18480
   ggttcgagga agtacggcgc cgccagcgcg ctactgcccg aatatgccct acatccttcc 18540
   attgcgccta cccccggcta tcgtggctac acctaccgcc ccagaagacg agcaactacc 18600
   cgacgccgaa ccaccactgg aacccgccgc cgccgtcgcc gtcgccagcc cgtgctggcc 18660
   ccgatttccg tgcgcagggt ggctcgcgaa ggaggcagga ccctggtgct gccaacagcg 18720
   cgctaccacc ccagcatcgt ttaaaagccg gtctttgtgg ttcttgcaga tatggccctc 18780
   acctgccgcc tccgtttccc ggtgccggga ttccgaggaa gaatgcaccg taggaggggc 18840
   atggccggcc acggcctgac gggcggcatg cgtcgtgcgc accaccggcg gcggcgcgcg 18900
   tcgcaccgtc gcatgcgcgg cggtatcctg cccctcctta ttccactgat cgccgcggcg 18960
   attggcgccg tgcccggaat tgcatccgtg gccttgcagg cgcagagaca ctgattaaaa 19020
   acaagttgca tgtggaaaaa tcaaaataaa aagtctggac tctcacgctc gcttggtcct 19080
   gtaactattt tgtagaatgg aagacatcaa ctttgcgtct ctggccccgc gacacggctc 19140
   gcgcccgttc atgggaaact ggcaagatat cggcaccagc aatatgagcg gtggcgcctt 19200
   cagctggggc tcgctgtgga gcggcattaa aaatttcggt tccaccgtta agaactatgg 19260
   cagcaaggcc tggaacagca gcacaggcca gatgctgagg gataagttga aagagcaaaa 19320
   tttccaacaa aaggtggtag atggcctggc ctctggcatt agcggggtgg tggacctggc 19380
   caaccaggca gtgcaaaata agattaacag taagcttgat ccccgccctc ccgtagagga 19440
   gcctccaccg gccgtggaga cagtgtctcc agaggggcgt ggcgaaaagc gtccgcgccc 19500
   cgacagggaa gaaactctgg tgacgcaaat agacgagcct ccctcgtacg aggaggcact 19560
   aaagcaaggc ctgcccacca cccgtcccat cgcgcccatg gctaccggag tgctgggcca 19620
   gcacacaccc gtaacgctgg acctgcctcc ccccgccgac acccagcaga aacctgtgct 19680
   gccaggcccg accgccgttg ttgtaacccg tcctagccgc gcgtccctgc gccgcgccgc 19740
   cagcggtccg cgatcgttgc ggcccgtagc cagtggcaac tggcaaagca cactgaacag 19800
   catcgtgggt ctgggggtgc aatccctgaa gcgccgacga tgcttctgaa tagctaacgt 19860
   gtcgtatgtg tgtcatgtat gcgtccatgt cgccgccaga ggagctgctg agccgccgcg 19920
   cgcccgcttt ccaagatggc taccccttcg atgatgccgc agtggtctta catgcacatc 19980
   tcgggccagg acgcctcgga gtacctgagc cccgggctgg tgcagtttgc ccgcgccacc 20040
   gagacgtact tcagcctgaa taacaagttt agaaacccca cggtggcgcc tacgcacgac 20100
   gtgaccacag accggtccca gcgtttgacg ctgcggttca tccctgtgga ccgtgaggat 20160
   actgcgtact cgtacaaggc gcggttcacc ctagctgtgg gtgataaccg tgtgctggac 20220
   atggcttcca cgtactttga catccgcggc gtgctggaca ggggccctac ttttaagccc 20280
   tactctggca ctgcctacaa cgccctggct cccaagggtg ccccaaatcc ttgcgaatgg 20340
   gatgaagctg ctactgctct tgaaataaac ctagaagaag aggacgatga caacgaagac 20400
   gaagtagacg agcaagctga gcagcaaaaa actcacgtat ttgggcaggc gccttattct 20460
   ggtataaata ttacaaagga gggtattcaa ataggtgtcg aaggtcaaac acctaaatat 20520
   gccgataaaa catttcaacc tgaacctcaa ataggagaat ctcagtggta cgaaactgaa 20580
   attaatcatg cagctgggag agtccttaaa aagactaccc caatgaaacc atgttacggt 20640
   tcatatgcaa aacccacaaa tgaaaatgga gggcaaggca ttcttgtaaa gcaacaaaat 20700
   ggaaagctag aaagtcaagt ggaaatgcaa tttttctcaa ctactgaggc gaccgcaggc 20760
   aatggtgata acttgactcc taaagtggta ttgtacagtg aagatgtaga tatagaaacc 20820
   ccagacactc atatttctta catgcccact attaaggaag gtaactcacg agaactaatg 20880
   ggccaacaat ctatgcccaa caggcctaat tacattgctt ttagggacaa ttttattggt 20940
   ctaatgtatt acaacagcac gggtaatatg ggtgttctgg cgggccaagc atcgcagttg 21000
   aatgctgttg tagatttgca agacagaaac acagagcttt cataccagct tttgcttgat 21060
   tccattggtg atagaaccag gtacttttct atgtggaatc aggctgttga cagctatgat 21120
   ccagatgtta gaattattga aaatcatgga actgaagatg aacttccaaa ttactgcttt 21180
   ccactgggag gtgtgattaa tacagagact cttaccaagg taaaacctaa aacaggtcag 21240
   gaaaatggat gggaaaaaga tgctacagaa ttttcagata aaaatgaaat aagagttgga 21300
   aataattttg ccatggaaat caatctaaat gccaacctgt ggagaaattt cctgtactcc 21360
   aacatagcgc tgtatttgcc cgacaagcta aagtacagtc cttccaacgt aaaaatttct 21420
   gataacccaa acacctacga ctacatgaac aagcgagtgg tggctcccgg gttagtggac 21480
   tgctacatta accttggagc acgctggtcc cttgactata tggacaacgt caacccattt 21540
   aaccaccacc gcaatgctgg cctgcgctac cgctcaatgt tgctgggcaa tggtcgctat 21600
   gtgcccttcc acatccaggt gcctcagaag ttctttgcca ttaaaaacct ccttctcctg 21660
   ccgggctcat acacctacga gtggaacttc aggaaggatg ttaacatggt tctgcagagc 21720
   tccctaggaa atgacctaag ggttgacgga gccagcatta agtttgatag catttgcctt 21780
   tacgccacct tcttccccat ggcccacaac accgcctcca cgcttgaggc catgcttaga 21840
   aacgacacca acgaccagtc ctttaacgac tatctctccg ccgccaacat gctctaccct 21900
   atacccgcca acgctaccaa cgtgcccata tccatcccct cccgcaactg ggcggctttc 21960
   cgcggctggg ccttcacgcg ccttaagact aaggaaaccc catcactggg ctcgggctac 22020
   gacccttatt acacctactc tggctctata ccctacctag atggaacctt ttacctcaac 22080
   cacaccttta agaaggtggc cattaccttt gactcttctg tcagctggcc tggcaatgac 22140
   cgcctgctta cccccaacga gtttgaaatt aagcgctcag ttgacgggga gggttacaac 22200
   gttgcccagt gtaacatgac caaagactgg ttcctggtac aaatgctagc taactacaac 22260
   attggctacc agggcttcta tatcccagag agctacaagg accgcatgta ctccttcttt 22320
   agaaacttcc agcccatgag ccgtcaggtg gtggatgata ctaaatacaa ggactaccaa 22380
   caggtgggca tcctacacca acacaacaac tctggatttg ttggctacct tgcccccacc 22440
   atgcgcgaag gacaggccta ccctgctaac ttcccctatc cgcttatagg caagaccgca 22500
   gttgacagca ttacccagaa aaagtttctt tgcgatcgca ccctttggcg catcccattc 22560
   tccagtaact ttatgtccat gggcgcactc acagacctgg gccaaaacct tctctacgcc 22620
   aactccgccc acgcgctaga catgactttt gaggtggatc ccatggacga gcccaccctt 22680
   ctttatgttt tgtttgaagt ctttgacgtg gtccgtgtgc accggccgca ccgcggcgtc 22740
   atcgaaaccg tgtacctgcg cacgcccttc tcggccggca acgccacaac ataaagaagc 22800
   aagcaacatc aacaacagct gccgccatgg gctccagtga gcaggaactg aaagccattg 22860
   tcaaagatct tggttgtggg ccatattttt tgggcaccta tgacaagcgc tttccaggct 22920
   ttgtttctcc acacaagctc gcctgcgcca tagtcaatac ggccggtcgc gagactgggg 22980
   gcgtacactg gatggccttt gcctggaacc cgcactcaaa aacatgctac ctctttgagc 23040
   cctttggctt ttctgaccag cgactcaagc aggtttacca gtttgagtac gagtcactcc 23100
   tgcgccgtag cgccattgct tcttcccccg accgctgtat aacgctggaa aagtccaccc 23160
   aaagcgtaca ggggcccaac tcggccgcct gtggactatt ctgctgcatg tttctccacg 23220
   cctttgccaa ctggccccaa actcccatgg atcacaaccc caccatgaac cttattaccg 23280
   gggtacccaa ctccatgctc aacagtcccc aggtacagcc caccctgcgt cgcaaccagg 23340
   aacagctcta cagcttcctg gagcgccact cgccctactt ccgcagccac agtgcgcaga 23400
   ttaggagcgc cacttctttt tgtcacttga aaaacatgta aaaataatgt actagagaca 23460
   ctttcaataa aggcaaatgc ttttatttgt acactctcgg gtgattattt acccccaccc 23520
   ttgccgtctg cgccgtttaa aaatcaaagg ggttctgccg cgcatcgcta tgcgccactg 23580
   gcagggacac gttgcgatac tggtgtttag tgctccactt aaactcaggc acaaccatcc 23640
   gcggcagctc ggtgaagttt tcactccaca ggctgcgcac catcaccaac gcgtttagca 23700
   ggtcgggcgc cgatatcttg aagtcgcagt tggggcctcc gccctgcgcg cgcgagttgc 23760
   gatacacagg gttgcagcac tggaacacta tcagcgccgg gtggtgcacg ctggccagca 23820
   cgctcttgtc ggagatcaga tccgcgtcca ggtcctccgc gttgctcagg gcgaacggag 23880
   tcaactttgg tagctgcctt cccaaaaagg gcgcgtgccc aggctttgag ttgcactcgc 23940
   accgtagtgg catcaaaagg tgaccgtgcc cggtctgggc gttaggatac agcgcctgca 24000
   taaaagcctt gatctgctta aaagccacct gagcctttgc gccttcagag aagaacatgc 24060
   cgcaagactt gccggaaaac tgattggccg gacaggccgc gtcgtgcacg cagcaccttg 24120
   cgtcggtgtt ggagatctgc accacatttc ggccccaccg gttcttcacg atcttggcct 24180
   tgctagactg ctccttcagc gcgcgctgcc cgttttcgct cgtcacatcc atttcaatca 24240
   cgtgctcctt atttatcata atgcttccgt gtagacactt aagctcgcct tcgatctcag 24300
   cgcagcggtg cagccacaac gcgcagcccg tgggctcgtg atgcttgtag gtcacctctg 24360
   caaacgactg caggtacgcc tgcaggaatc gccccatcat cgtcacaaag gtcttgttgc 24420
   tggtgaaggt cagctgcaac ccgcggtgct cctcgttcag ccaggtcttg catacggccg 24480
   ccagagcttc cacttggtca ggcagtagtt tgaagttcgc ctttagatcg ttatccacgt 24540
   ggtacttgtc catcagcgcg cgcgcagcct ccatgccctt ctcccacgca gacacgatcg 24600
   gcacactcag cgggttcatc accgtaattt cactttccgc ttcgctgggc tcttcctctt 24660
   cctcttgcgt ccgcatacca cgcgccactg ggtcgtcttc attcagccgc cgcactgtgc 24720
   gcttacctcc tttgccatgc ttgattagca ccggtgggtt gctgaaaccc accatttgta 24780
   gcgccacatc ttctctttct tcctcgctgt ccacgattac ctctggtgat ggcgggcgct 24840
   cgggcttggg agaagggcgc ttctttttct tcttgggcgc aatggccaaa tccgccgccg 24900
   aggtcgatgg ccgcgggctg ggtgtgcgcg gcaccagcgc gtcttgtgat gagtcttcct 24960
   cgtcctcgga ctcgatacgc cgcctcatcc gcttttttgg gggcgcccgg ggaggcggcg 25020
   gcgacgggga cggggacgac acgtcctcca tggttggggg acgtcgcgcc gcaccgcgtc 25080
   cgcgctcggg ggtggtttcg cgctgctcct cttcccgact ggccatttcc ttctcctata 25140
   ggcagaaaaa gatcatggag tcagtcgaga agaaggacag cctaaccgcc ccctctgagt 25200
   tcgccaccac cgcctccacc gatgccgcca acgcgcctac caccttcccc gtcgaggcac 25260
   ccccgcttga ggaggaggaa gtgattatcg agcaggaccc aggttttgta agcgaagacg 25320
   acgaggaccg ctcagtacca acagaggata aaaagcaaga ccaggacaac gcagaggcaa 25380
   acgaggaaca agtcgggcgg ggggacgaaa ggcatggcga ctacctagat gtgggagacg 25440
   acgtgctgtt gaagcatctg cagcgccagt gcgccattat ctgcgacgcg ttgcaagagc 25500
   gcagcgatgt gcccctcgcc atagcggatg tcagccttgc ctacgaacgc cacctattct 25560
   caccgcgcgt accccccaaa cgccaagaaa acggcacatg cgagcccaac ccgcgcctca 25620
   acttctaccc cgtatttgcc gtgccagagg tgcttgccac ctatcacatc tttttccaaa 25680
   actgcaagat acccctatcc tgccgtgcca accgcagccg agcggacaag cagctggcct 25740
   tgcggcaggg cgctgtcata cctgatatcg cctcgctcaa cgaagtgcca aaaatctttg 25800
   agggtcttgg acgcgacgag aagcgcgcgg caaacgctct gcaacaggaa aacagcgaaa 25860
   atgaaagtca ctctggagtg ttggtggaac tcgagggtga caacgcgcgc ctagccgtac 25920
   taaaacgcag catcgaggtc acccactttg cctacccggc acttaaccta ccccccaagg 25980
   tcatgagcac agtcatgagt gagctgatcg tgcgccgtgc gcagcccctg gagagggatg 26040
   caaatttgca agaacaaaca gaggagggcc tacccgcagt tggcgacgag cagctagcgc 26100
   gctggcttca aacgcgcgag cctgccgact tggaggagcg acgcaaacta atgatggccg 26160
   cagtgctcgt taccgtggag cttgagtgca tgcagcggtt ctttgctgac ccggagatgc 26220
   agcgcaagct agaggaaaca ttgcactaca cctttcgaca gggctacgta cgccaggcct 26280
   gcaagatctc caacgtggag ctctgcaacc tggtctccta ccttggaatt ttgcacgaaa 26340
   accgccttgg gcaaaacgtg cttcattcca cgctcaaggg cgaggcgcgc cgcgactacg 26400
   tccgcgactg cgtttactta tttctatgct acacctggca gacggccatg ggcgtttggc 26460
   agcagtgctt ggaggagtgc aacctcaagg agctgcagaa actgctaaag caaaacttga 26520
   aggacctatg gacggccttc aacgagcgct ccgtggccgc gcacctggcg gacatcattt 26580
   tccccgaacg cctgcttaaa accctgcaac agggtctgcc agacttcacc agtcaaagca 26640
   tgttgcagaa ctttaggaac tttatcctag agcgctcagg aatcttgccc gccacctgct 26700
   gtgcacttcc tagcgacttt gtgcccatta agtaccgcga atgccctccg ccgctttggg 26760
   gccactgcta ccttctgcag ctagccaact accttgccta ccactctgac ataatggaag 26820
   acgtgagcgg tgacggtcta ctggagtgtc actgtcgctg caacctatgc accccgcacc 26880
   gctccctggt ttgcaattcg cagctgctta acgaaagtca aattatcggt acctttgagc 26940
   tgcagggtcc ctcgcctgac gaaaagtccg cggctccggg gttgaaactc actccggggc 27000
   tgtggacgtc ggcttacctt cgcaaatttg tacctgagga ctaccacgcc cacgagatta 27060
   ggttctacga agaccaatcc cgcccgccaa atgcggagct taccgcctgc gtcattaccc 27120
   agggccacat tcttggccaa ttgcaagcca tcaacaaagc ccgccaagag tttctgctac 27180
   gaaagggacg gggggtttac ttggaccccc agtccggcga ggagctcaac ccaatccccc 27240
   cgccgccgca gccctatcag cagcagccgc gggcccttgc ttcccaggat ggcacccaaa 27300
   aagaagctgc agctgccgcc gccacccacg gacgaggagg aatactggga cagtcaggca 27360
   gaggaggttt tggacgagga ggaggaggac atgatggaag actgggagag cctagacgag 27420
   gaagcttccg aggtcgaaga ggtgtcagac gaaacaccgt caccctcggt cgcattcccc 27480
   tcgccggcgc cccagaaatc ggcaaccggt tccagcatgg ctacaacctc cgctcctcag 27540
   gcgccgccgg cactgcccgt tcgccgaccc aaccgtagat gggacaccac tggaaccagg 27600
   gccggtaagt ccaagcagcc gccgccgtta gcccaagagc aacaacagcg ccaaggctac 27660
   cgctcatggc gcgggcacaa gaacgccata gttgcttgct tgcaagactg tgggggcaac 27720
   atctccttcg cccgccgctt tcttctctac catcacggcg tggccttccc ccgtaacatc 27780
   ctgcattact accgtcatct ctacagccca tactgcaccg gcggcagcgg cagcggcagc 27840
   aacagcagcg gccacacaga agcaaaggcg accggatagc aagactctga caaagcccaa 27900
   gaaatccaca gcggcggcag cagcaggagg aggagcgctg cgtctggcgc ccaacgaacc 27960
   cgtatcgacc cgcgagctta gaaacaggat ttttcccact ctgtatgcta tatttcaaca 28020
   gagcaggggc caagaacaag agctgaaaat aaaaaacagg tctctgcgat ccctcacccg 28080
   cagctgcctg tatcacaaaa gcgaagatca gcttcggcgc acgctggaag acgcggaggc 28140
   tctcttcagt aaatactgcg cgctgactct taaggactag tttcgcgccc tttctcaaat 28200
   ttaagcgcga aaactacgtc atctccagcg gccacacccg gcgccagcac ctgtcgtcag 28260
   cgccattatg agcaaggaaa ttcccacgcc ctacatgtgg agttaccagc cacaaatggg 28320
   acttgcggct ggagctgccc aagactactc aacccgaata aactacatga gcgcgggacc 28380
   ccacatgata tcccgggtca acggaatccg cgcccaccga aaccgaattc tcttggaaca 28440
   ggcggctatt accaccacac ctcgtaataa ccttaatccc cgtagttggc ccgctgccct 28500
   ggtgtaccag gaaagtcccg ctcccaccac tgtggtactt cccagagacg cccaggccga 28560
   agttcagatg actaactcag gggcgcagct tgcgggcggc tttcgtcaca gggtgcggtc 28620
   gcccgggcag ggtataactc acctgacaat cagagggcga ggtattcagc tcaacgacga 28680
   gtcggtgagc tcctcgcttg gtctccgtcc ggacgggaca tttcagatcg gcggcgccgg 28740
   ccgtccttca ttcacgcctc gtcaggcaat cctaactctg cagacctcgt cctctgagcc 28800
   gcgctctgga ggcattggaa ctctgcaatt tattgaggag tttgtgccat cggtctactt 28860
   taacccctta tcgggacctc ccggccacta tccggatcaa tttattccta actttgacgc 28920
   ggtaaaggac tcggcggacg gctacgactg aatgttaagt ggagaggcag agcaactgcg 28980
   cctgaaacac ctggtccact gtcgccgcca caagtgcttt gcccgcgact ccggtgagtt 29040
   ttgctacttt gaattgcccg aggatcatat cgagggcccg gcgcacggcg tccggcttac 29100
   cgcccaggga gagcttgccc gtagcctgat tcgggagttt acccagcgcc ccctgctagt 29160
   tgagcgggac aggggaccct gtgttctcac tgtgatttgc aactgtccta accttggatt 29220
   acatcaagat cctctagtta taactagagt acccggggat cttattccct ttaactaata 29280
   aaaaaaaata ataaagcatc acttacttaa aatcagttag caaatttctg tccagtttat 29340
   tcagcagcac ctccttgccc tcctcccagc tctggtattg cagcttcctc ctggctgcaa 29400
   actttctcca caatctaaat ggaatgtcag tttcctcctg ttcctgtcca tccgcaccca 29460
   ctatcttcat gttgttgcag atgaagcgcg caagaccgtc tgaagatacc ttcaaccccg 29520
   tgtatccata tgacacggaa accggtcctc caactgtgcc ttttcttact cctccctttg 29580
   tatcccccaa tgggtttcaa gagagtcccc ctggggtact ctctttgcgc ctatccgaac 29640
   ctctagttac ctccaatggc atgcttgcgc tcaaaatggg caacggcctc tctctggacg 29700
   aggccggcaa ccttacctcc caaaatgtaa ccactgtgag cccacctctc aaaaaaacca 29760
   agtcaaacat aaacctggaa atatctgcac ccctcacagt tacctcagaa gccctaactg 29820
   tggctgccgc cgcacctcta atggtcgcgg gcaacacact caccatgcaa tcacaggccc 29880
   cgctaaccgt gcacgactcc aaacttagca ttgccaccca aggacccctc acagtgtcag 29940
   aaggaaagct agccctgcaa acatcaggcc ccctcaccac caccgatagc agtaccctta 30000
   ctatcactgc ctcaccccct ctaactactg ccactggtag cttgggcatt gacttgaaag 30060
   agcccattta tacacaaaat ggaaaactag gactaaagta cggggctcct ttgcatgtaa 30120
   cagacgacct aaacactttg accgtagcaa ctggtccagg tgtgactatt aataatactt 30180
   ccttgcaaac taaagttact ggagccttgg gttttgattc acaaggcaat atgcaactta 30240
   atgtagcagg aggactaagg attgattctc aaaacagacg ccttatactt gatgttagtt 30300
   atccgtttga tgctcaaaac caactaaatc taagactagg acagggccct ctttttataa 30360
   actcagccca caacttggat attaactaca acaaaggcct ttacttgttt acagcttcaa 30420
   acaattccaa aaagcttgag gttaacctaa gcactgccaa ggggttgatg tttgacgcta 30480
   cagccatagc cattaatgca ggagatgggc ttgaatttgg ttcacctaat gcaccaaaca 30540
   caaatcccct caaaacaaaa attggccatg gcctagaatt tgattcaaac aaggctatgg 30600
   ttcctaaact aggaactggc cttagttttg acagcacagg tgccattaca gtaggaaaca 30660
   aaaataatga taagctaact ttgtggacca caccagctcc atctcctaac tgtagactaa 30720
   atgcagagaa agatgctaaa ctcactttgg tcttaacaaa atgtggcagt caaatacttg 30780
   ctacagtttc agttttggct gttaaaggca gtttggctcc aatatctgga acagttcaaa 30840
   gtgctcatct tattataaga tttgacgaaa atggagtgct actaaacaat tccttcctgg 30900
   acccagaata ttggaacttt agaaatggag atcttactga aggcacagcc tatacaaacg 30960
   ctgttggatt tatgcctaac ctatcagctt atccaaaatc tcacggtaaa actgccaaaa 31020
   gtaacattgt cagtcaagtt tacttaaacg gagacaaaac taaacctgta acactaacca 31080
   ttacactaaa cggtacacag gaaacaggag acacaactcc aagtgcatac tctatgtcat 31140
   tttcatggga ctggtctggc cacaactaca ttaatgaaat atttgccaca tcctcttaca 31200
   ctttttcata cattgcccaa gaataaagaa tcgtttgtgt tatgtttcaa cgtgtttatt 31260
   tttcaattgc agaaaatttc aagtcatttt tcattcagta gtatagcccc accaccacat 31320
   agcttataca gatcaccgta ccttaatcaa actcacagaa ccctagtatt caacctgcca 31380
   cctccctccc aacacacaga gtacacagtc ctttctcccc ggctggcctt aaaaagcatc 31440
   atatcatggg taacagacat attcttaggt gttatattcc acacggtttc ctgtcgagcc 31500
   aaacgctcat cagtgatatt aataaactcc ccgggcagct cacttaagtt catgtcgctg 31560
   tccagctgct gagccacagg ctgctgtcca acttgcggtt gcttaacggg cggcgaagga 31620
   gaagtccacg cctacatggg ggtagagtca taatcgtgca tcaggatagg gcggtggtgc 31680
   tgcagcagcg cgcgaataaa ctgctgccgc cgccgctccg tcctgcagga atacaacatg 31740
   gcagtggtct cctcagcgat gattcgcacc gcccgcagca taaggcgcct tgtcctccgg 31800
   gcacagcagc gcaccctgat ctcacttaaa tcagcacagt aactgcagca cagcaccaca 31860
   atattgttca aaatcccaca gtgcaaggcg ctgtatccaa agctcatggc ggggaccaca 31920
   gaacccacgt ggccatcata ccacaagcgc aggtagatta agtggcgacc cctcataaac 31980
   acgctggaca taaacattac ctcttttggc atgttgtaat tcaccacctc ccggtaccat 32040
   ataaacctct gattaaacat ggcgccatcc accaccatcc taaaccagct ggccaaaacc 32100
   tgcccgccgg ctatacactg cagggaaccg ggactggaac aatgacagtg gagagcccag 32160
   gactcgtaac catggatcat catgctcgtc atgatatcaa tgttggcaca acacaggcac 32220
   acgtgcatac acttcctcag gattacaagc tcctcccgcg ttagaaccat atcccaggga 32280
   acaacccatt cctgaatcag cgtaaatccc acactgcagg gaagacctcg cacgtaactc 32340
   acgttgtgca ttgtcaaagt gttacattcg ggcagcagcg gatgatcctc cagtatggta 32400
   gcgcgggttt ctgtctcaaa aggaggtaga cgatccctac tgtacggagt gcgccgagac 32460
   aaccgagatc gtgttggtcg tagtgtcatg ccaaatggaa cgccggacgt agtcatattt 32520
   cctgaagcaa aaccaggtgc gggcgtgaca aacagatctg cgtctccggt ctcgccgctt 32580
   agatcgctct gtgtagtagt tgtagtatat ccactctctc aaagcatcca ggcgccccct 32640
   ggcttcgggt tctatgtaaa ctccttcatg cgccgctgcc ctgataacat ccaccaccgc 32700
   agaataagcc acacccagcc aacctacaca ttcgttctgc gagtcacaca cgggaggagc 32760
   gggaagagct ggaagaacca tgtttttttt tttattccaa aagattatcc aaaacctcaa 32820
   aatgaagatc tattaagtga acgcgctccc ctccggtggc gtggtcaaac tctacagcca 32880
   aagaacagat aatggcattt gtaagatgtt gcacaatggc ttccaaaagg caaacggccc 32940
   tcacgtccaa gtggacgtaa aggctaaacc cttcagggtg aatctcctct ataaacattc 33000
   cagcaccttc aaccatgccc aaataattct catctcgcca ccttctcaat atatctctaa 33060
   gcaaatcccg aatattaagt ccggccattg taaaaatctg ctccagagcg ccctccacct 33120
   tcagcctcaa gcagcgaatc atgattgcaa aaattcaggt tcctcacaga cctgtataag 33180
   attcaaaagc ggaacattaa caaaaatacc gcgatcccgt aggtcccttc gcagggccag 33240
   ctgaacataa tcgtgcaggt ctgcacggac cagcgcggcc acttccccgc caggaacctt 33300
   gacaaaagaa cccacactga ttatgacacg catactcgga gctatgctaa ccagcgtagc 33360
   cccgatgtaa gctttgttgc atgggcggcg atataaaatg caaggtgctg ctcaaaaaat 33420
   caggcaaagc ctcgcgcaaa aaagaaagca catcgtagtc atgctcatgc agataaaggc 33480
   aggtaagctc cggaaccacc acagaaaaag acaccatttt tctctcaaac atgtctgcgg 33540
   gtttctgcat aaacacaaaa taaaataaca aaaaaacatt taaacattag aagcctgtct 33600
   tacaacagga aaaacaaccc ttataagcat aagacggact acggccatgc cggcgtgacc 33660
   gtaaaaaaac tggtcaccgt gattaaaaag caccaccgac agctcctcgg tcatgtccgg 33720
   agtcataatg taagactcgg taaacacatc aggttgattc atcggtcagt gctaaaaagc 33780
   gaccgaaata gcccggggga atacataccc gcaggcgtag agacaacatt acagccccca 33840
   taggaggtat aacaaaatta ataggagaga aaaacacata aacacctgaa aaaccctcct 33900
   gcctaggcaa aatagcaccc tcccgctcca gaacaacata cagcgcttca cagcggcagc 33960
   ctaacagtca gccttaccag taaaaaagaa aacctattaa aaaaacacca ctcgacacgg 34020
   caccagctca atcagtcaca gtgtaaaaaa gggccaagtg cagagcgagt atatatagga 34080
   ctaaaaaatg acgtaacggt taaagtccac aaaaaacacc cagaaaaccg cacgcgaacc 34140
   tacgcccaga aacgaaagcc aaaaaaccca caacttcctc aaatcgtcac ttccgttttc 34200
   ccacgttacg taacttccca ttttaagaaa actacaattc ccaacacata caagttactc 34260
   cgccctaaaa cctacgtcac ccgccccgtt cccacgcccc gcgccacgtc acaaactcca 34320
   ccccctcatt atcatattgg cttcaatcca aaataaggta tattattgat gatnnnnntt 34380
   aat                                                               34383

## Description

### BACKGROUND OF THE INVENTION

Several different methods have been proposed to prevent influenza infection, but most of these methods use old technologies administered via injection. This can be difficult to accomplish in a pandemic where normal health care has been disrupted. An oral delivery vaccine that can be distributed without qualified medical personnel would have a significant advantage.

The H1N1 pandemic viruses of 2009 target different cell populations than normal influenza. These viruses infect intestinal epithelial cells, as well as lung epithelial cells. Patients infected with H1N1 reported diarrhea and stomach illness along with the usual influenza symptoms.

The present invention provides an oral immunization with substantial benefits in protecting against H1N1 infection. A strong mucosal immune response is generated, including in the intestine, as evidenced by the strong IgA antibody responses against HA in fecal pellets. The oral H1N1 vaccine is also shown to be protective against H1N1 in a direct challenge in vivo.

The prior art includes WO2007100908. Example 4 thereof discloses delivery of an adenoviral vector encoding an influenza A/PR/8/34 HA protein in combination with a poly I:C TLR-3 antagonist. Example 11 thereof discloses an adenoviral vector with a promoter controlling expression of HA of the influenza strain A/Indo/5/2005 with a downstream sequence encoding a double stranded RNA TLR-3 agonist. This example also discloses an adenoviral vector with a first promoter controlling expression of a double stranded RNA TLR-3 agonist, and a second promoter controlling expression of HA of the influenza strain A/Indo/5/2005.

### BRIEF SUMMARY OF THE INVENTION

The invention provides rapid-response and easily scalable compositions for vaccination against pandemic strains of H1N1 influenza. Upon isolation of the strain, a gene-based viral vaccine according to the invention can be prepared and tested for safety and efficacy within weeks.

The invention provides chimeric adenoviral expression vectors for protection against H1N1 influenza, said vector comprising an expression cassette comprising (a) a first promoter operably linked to a nucleic acid encoding a toll-like receptor-3 (TLR-3) agonist, wherein the TLR-3 agonist is a double stranded RNA (dsRNA); and (b) a second promoter operably linked to a nucleic acid encoding haemagglutinin polypeptide from A/CA/04/2009 H1N1 influenza. In some embodiments, the dsRNA is selected from short hairpin RNA (shRNA), viral dsRNA, and siRNA. In some embodiments, the sequence encoding the dsRNA comprises a sequence having substantial identity to a sequence selected from the group consisting of SEQ ID NO:1, SEQ ID NO:2, SEQ ID NO:3, SEQ ID NO:4, SEQ ID NO:5, and SEQ ID NO:6 (SEQ ID NOs:1-6). In some embodiments, the sequence encoding the dsRNA comprises a sequence selected from the group consisting of SEQ ID NOs:1-6.

In some aspects of the disclosure described herein, the polypeptide from H1N1 is a surface antigen of H1N1 or an antigenic fragment thereof. In some aspects of the disclosure described herein, the H1N1 polypeptide is selected from the group consisting of HA, NA, MP, and NP, and antigenic fragments thereof. The H1N1 influenza is from the strain A/CA/04/2009.

In some embodiments, the first promoter and the second promoter are the same. In some embodiments, the first promoter and second promoter are different. The promoter can be constitutive or inducible, and can be a viral or mammalian gene promoter, *e.g.,* CMV or SV40.

In some embodiments described herein, the viral expression vector is substantially identical to SEQ ID NO:7, *e.g.,* with at least 90,92, 95, 96, 98, or 99% identity to SEQ ID NO:7, or to the expression cassette of SEQ ID NO:7 (nucleotide positions 603 to 4194). In some embodiments described herein, the viral expression vector comprises SEQ ID NO:7 or the expression cassette of SEQ ID NO:7. In some aspects of the disclosure described herein, the viral expression vector is substantially identical to SEQ ID NO:7, but encodes a different H1N1 antigenic polypeptide.

In some embodiments, the invention provides pharmaceutical compositions for protection against H1N1 influenza, said compositions comprising the chimeric viral vector of the invention and a pharmaceutically acceptable carrier. In some embodiments, the pharmaceutical composition is formulated for non-parenteral delivery (administration), e.g., oral, mucosal, or intranasal delivery. In some embodiments, the pharmaceutical composition is formulated for oral delivery.

In some embodiments, the invention provides an oral delivery pharmaceutical composition comprising the chimeric viral vector of the invention and a pharmaceutically acceptable carrier..

The invention also provides an immunogenically effective amount of a chimeric adenoviral expression vector of the invention, or a composition of the invention, for use in a method for eliciting an immune response to pandemic H1N1 influenza in a mammal, wherein the immune response is directed against haemagglutinin polypeptide, and wherein the route of administration is selected from the group consisting of: oral, intranasal, and mucosal. In some embodiments, the administration is oral. In some embodiments, the mammal is a human. In some embodiments, the immune response is selected from the group consisting of a mucosal immune response, a systemic immune response, a humoral immune response, and a cellular immune response. In some embodiments, the immune response is a mucosal immune response. In some embodiments, the immune response can be detected in intestinal mucosa.

### BRIEF DESCRIPTION OF THE DRAWINGS

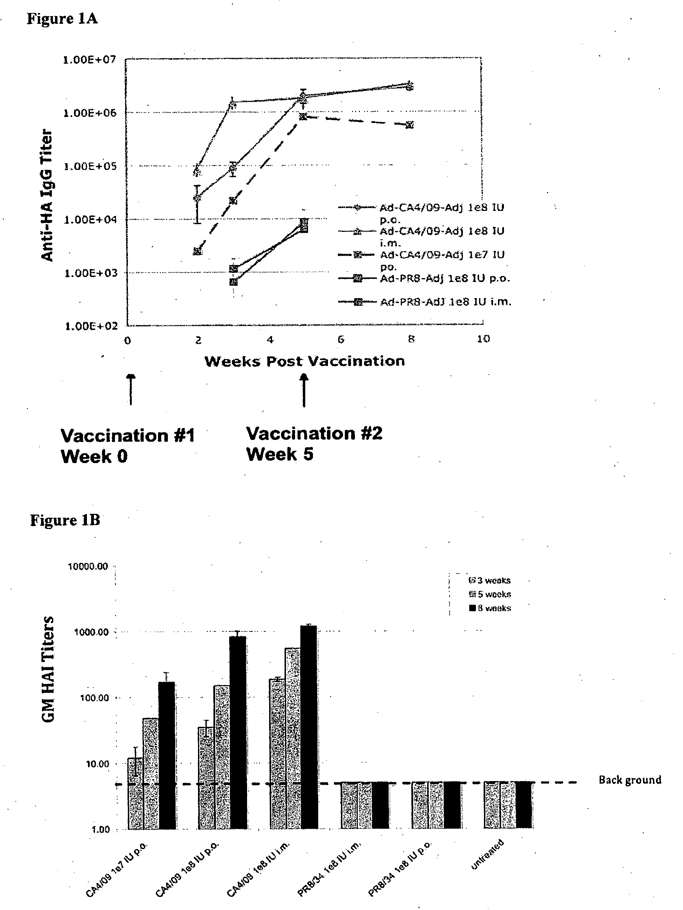

**Figure 1A****.** Systemic antibody titers for anti-influenza HA antibodies are comparable at 8 weeks for oral (p.o.) and intramuscular (i.m.) vaccination of DX1. Ad-CA/04/09-Adj was administered orally at two different doses (10⁷ IU or 10⁸ IU) or i.m. at 10⁸ IU. Results from a similar chimeric adenoviral expression vector encoding HA from a non-similar H1N1 are also shown (Ad-PR8-Adj).
**Figure 1B****.** Results from Hemagglutination Inhibition (HAI) at 3, 5, and 8 weeks post-vaccination confirm similar systemic antibody response to A/CA/04/2009 from oral and i.m. administration.

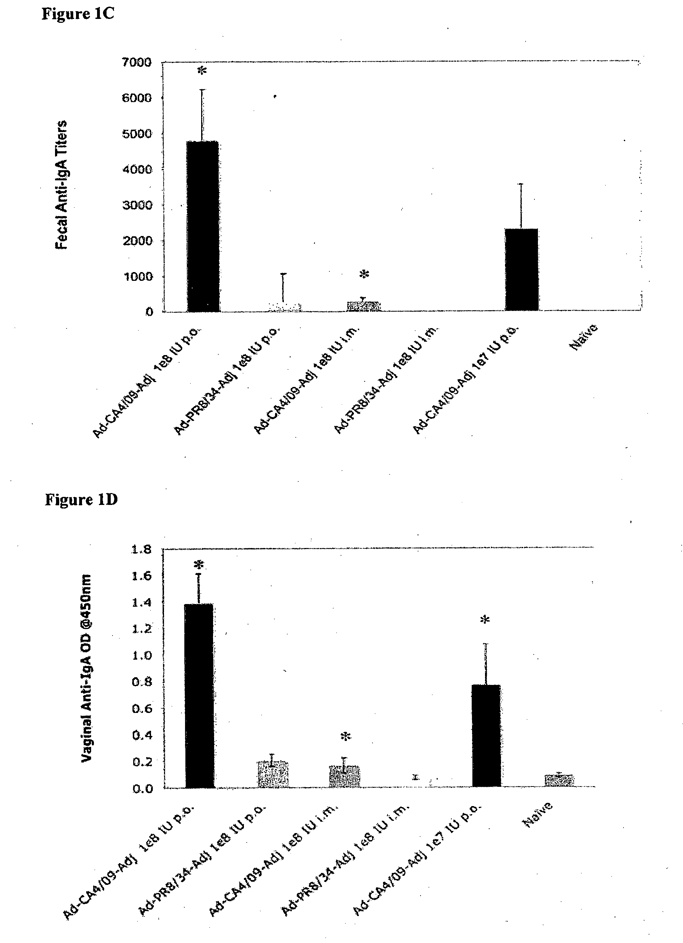

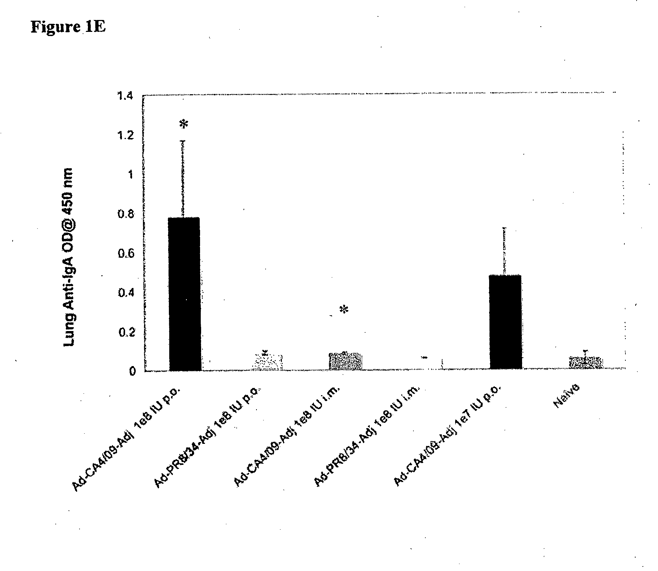

**Figure 1C, 1D****, and 1F.** Oral administration of DX1 vector vaccine provides superior mucosal antibody response compared to i.m. injection. Titers of antibodies specific to HA were measured in intestinal (fecal), vaginal, and lung mucosal tissues, at 8, 10, and 12 weeks post-administration, respectively. Results are shown in panels C, D, and E of Figure 1. Note that oral administration of DX1 vector provides significantly higher specific IgA titers than high dose i.m. injection. The 10-fold lower oral dose induces an equal or greater IgA response as well. *indicates P<0.05 calculated using Student's T-Test based on OD450 readings.

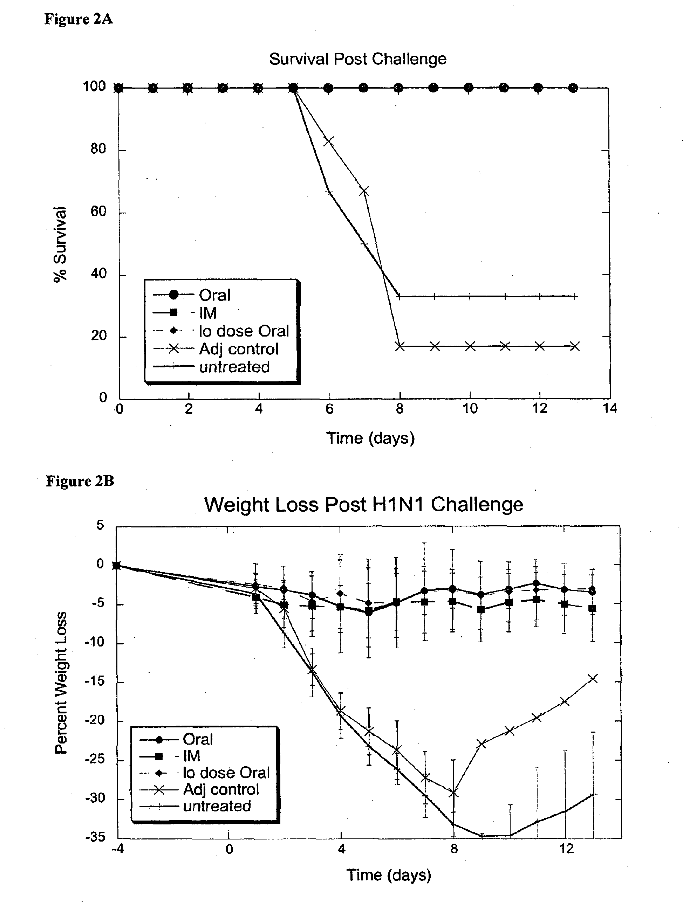

**Figure 2A and 2B****.** Low dose oral administration of DX1 vector provides comparable survival and weight maintenance data to high dose oral and i.m. administration. Figure 2A shows that 100% of DX1 vaccinated mice receiving a lethal H1N1 challenge survived. Similarly, Figure 2B shows minimal weight loss in all three groups. Untreated mice, or those vaccinated with adjuvant alone succumbed to the virus soon after challenge. Oral indicates 10⁸ IU DX1, IM indicates 10⁸ IU DX1, lo dose oral indicates 10⁷ IU DX1, Adj control indicates vector expressing dsRNA only.

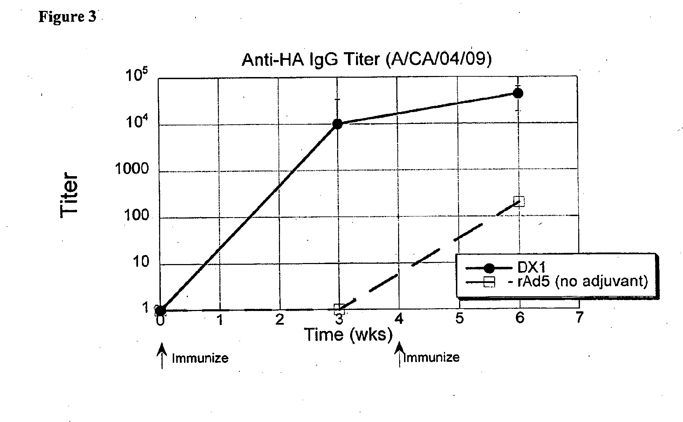

**Figure 3****.** Expression of dsRNA adjuvant improves antibody response to the H1N1 HA antigen. Plasma titers of antibodies specific for HA were measured following oral administration of DX1 and DX2 (vector lacking dsRNA encoding sequence).

### DETAILED DESCRIPTION OF THE INVENTION

### I. Introduction

The present invention provides novel and effective chimeric adeno viral vector vaccines that can be rapidly developed in response to an H1N1 viral pandemic. Once a pandemic strain is isolated, an antigen can be isolated and used to design a vaccine according to the present invention. Animal testing, to determine safety and tolerable dose ranges, as well as efficacy of the vaccine against the particular pandemic strain, can be accomplished on the order of weeks. The chimeric adeno viral vector vaccines of the invention comprise a sequence encoding antigen (haemagglutinin) as well as a sequence encoding a dsRNA adjuvant. The vaccine can be administered non-parenterally and at a low dose to elicit an immune response against the H1N1 antigen. The chimeric viral vectors elicit strong and effective immune responses specific for the H1N1 polypeptide antigen, particularly when administered via a non-parenteral route (*e.g.,* orally, intranasally, or mucosally).

The efficacy of the vector-encoded dsRNA TLR-3 agonist as an adjuvant is also surprising. Use of dsRNA as an adjuvant for viral vectors is counterintuitive considering that the major proposed utility of the dsRNA mimetic poly I:C is as an antiviral agent [Nemes, et al., Proc Soc Exp Biol Med. (1969) 132:776; Schafer, et al, Nature. (1970) 226:449; Fenje, et al, Nature (1970) 226:171.].

### II. Definitions

The term "chimeric" or "recombinant" as used herein with reference, *e.g.,* to a nucleic acid, protein, or vector, indicates that the nucleic acid, protein or vector, has been modified by the introduction of a heterologous nucleic acid or protein or the alteration of a native nucleic acid or protein. Thus, for example, chimeric and recombinant vectors include nucleic acid sequences that are not found within the native (non-chimeric or non-recombinant) form of the vector. A chimeric viral vector (or chimeric viral expression vector) refers to a viral vector comprising a nucleic acid sequence encoding a heterologous polypeptide.

An "expression vector" or "expression cassette" is a nucleic acid construct, generated recombinantly or synthetically, with a series of specified nucleic acid elements that permit transcription of a particular nucleic acid in a host cell. The expression vector can be part of a plasmid, virus, or nucleic acid fragment. Typically, the expression vector includes a nucleic acid to be transcribed operably linked to a promoter. An expression vector can include more than one expression units or coding sequences, which can be contiguous (*e.g.,* under control of the same promoter) or non-contiguous (*e.g*., under control of different promoter).

The terms "promoter" and "expression control sequence" refer to nucleic acid control sequences that direct transcription of a nucleic acid. As used herein, a promoter includes necessary nucleic acid sequences near the start site of transcription, such as, in the case of a polymerase II type promoter, a TATA element. A promoter also optionally includes distal enhancer or repressor elements, which can be located as much as several thousand base pairs from the start site of transcription. Promoters include constitutive and inducible promoters. A "constitutive" promoter is a promoter that is active under most environmental and developmental conditions. An "inducible" promoter is a promoter that is active under environmental or developmental regulation. The term "operably linked" refers to a functional linkage between a nucleic acid expression control sequence (such as a promoter, or array of transcription factor binding sites) and a second nucleic acid sequence, wherein the expression control sequence directs transcription of the nucleic acid corresponding to the second sequence.

The terms "TLR-3 agonist" or "Toll-like receptor 3 agonist" as used herein refers to a compound that binds and stimulates the TLR-3. TLR-3 agonists have been identified including double-stranded RNA, virally derived dsRNA, several chemically synthesized analogs to double-stranded RNA including polyinosine-polycytidylic acid (poly I:C) -polyadenylic-polyuridylic acid (poly A:U) and poly I:poly C, and antibodies (or cross-linking of antibodies) to TLR-3 that lead to IFN-beta production [Matsumoto, M, et al, Biochem Biophys Res Commun 24:1364 (2002), de Bouteiller, et al, J Biol Chem 18:38133-45 (2005)]. TLR-3 agonists also include dsRNA encoded by and expressed from a vector, *e.g.,* as described herein.

Double-stranded RNA (dsRNA) is RNA that includes two complementary strands, typically with a complementary stretch of at least 4, 5, 10, 12, 15, 20 or more ribonucleotides. The complementary stretch of a dsRNA can be between 5-10, 5-20, 5-30, 10-20, 10-100, 20-100, and higher. One of skill will appreciate that the complementary stretch need not be 100% complementary for the dsRNA to act as an effective TLR-3 agonist or adjuvant. A dsRNA molecule can comprise an intramolecular double-stranded RNA species (*e.g.,* short hairpin RNA or siRNA) and dsRNA species that comprise at least two separate single-stranded RNA molecules.

Polynucleotides can comprise a designated sequence (*i.e*., an H1N1 viral sequence that encodes a particular polypeptide or dsRNA or a portion thereof) or a variant of such a sequence that results in an immune response to the desired H1N1 virus or TLR-3 agonism, respectively. Polynucleotide variants may contain one or more substitutions, additions, deletions and/or insertions such that the biological activity of the encoded polypeptide is not diminished, relative to a polypeptide comprising the original sequence. Polynucleotide variants of a given dsRNA can contain one or more substitutions, additions, deletions and/or insertions such that the TLR-3 agonist activity of the encoded dsRNA is not diminished, relative to a dsRNA that does not contain the substitutions, additions, deletions and/or insertions. Variants preferably exhibit at least about 50%, 55%, 60%, 65%, 70%, 75%, 80%, 85%, 90%, 91%, 92%, 93%, 94%, 95%, 96%, 97%, 98%, 99%, to a polynucleotide sequence that encodes a native polypeptide or a portion thereof or to a polynucleotide sequence that encodes a dsRNA with TLR-3 agonist activity.

The term "subject" or "patient," for the purposes of the present invention, refers to a mammal that can be infected with H1N1 virus. Mammals include humans, as well as non-human mammals such as rodents (*e.g.,* mice and rats), rabbits, livestock animals (e.g., bovine, porcine, and ovine animals) and pets (*e.g.,* dogs and cats), and non-human primates.

The term "heterologous" when used with reference to portions of a nucleic acid indicates that the nucleic acid comprises two or more subsequences that are not found in the same relationship to each other in nature. For instance, the nucleic acid is typically recombinantly produced, having two or more sequences from unrelated genes arranged to make a new functional nucleic acid, *e.g.,* a promoter from one source and a coding region from another source. Similarly, a heterologous protein indicates that the protein comprises two or more subsequences that are not found in the same relationship to each other in nature (*e.g.,* a fusion protein).

The terms "nucleic acid," "oligonucleotide," "polynucleotide" and like terms are used interchangeably herein to refer to polymers of deoxyribonucleotides or ribonucleotides in either single- or double-stranded form. The term encompasses nucleic acids containing known nucleotide analogs or modified backbone residues or linkages, which are synthetic, naturally occurring, and non-naturally occurring, which have similar binding properties as the reference nucleic acid, and which are metabolized in a manner similar to the reference nucleotides. Examples of such analogs include, without limitation, phosphorothioates, phosphoramidates, methyl phosphonates, chiral-methyl phosphonates, 2-O-methyl ribonucleotides, peptide-nucleic acids (PNAs).

Unless otherwise indicated, a particular nucleic acid sequence also encompasses conservatively modified variants thereof (*e.g.,* degenerate codon substitutions) and complementary sequences, as well as the sequence explicitly indicated. Specifically, degenerate codon substitutions may be achieved by generating sequences in which the third position of one or more selected (or all) codons is substituted with mixed-base and/or deoxyinosine residues (Batzer et al., Nucleic Acid Res. 19:5081 (1991); Ohtsuka et al., J. Biol. Chem. 260:2605-2608 (1985); Rossolini et al., Mol. Cell. Probes 8:91-98 (1994)).

Antigen- refers to a protein or part of a polypeptide chain that can be recognized by T cell receptors and/or antibodies. Typically, antigens are derived from bacterial, viral, or fungal proteins, or from abnormal proteins expressed by cancer cells.

An "immunogenically effective dose or amount" of a composition of the present invention is an amount that elicits or modulates an immune response specific for the desired antigenic polypeptide. Immune responses include humoral immune responses and cell-mediated immune responses. An immunogenic composition can be used therapeutically or prophylactically to treat or prevent H1N1 infection.

The terms "dose" and "dosage" are used interchangeably herein. A dose refers to the amount of active ingredient given to an individual at each administration. The dose for the purposes of the present invention can be expressed in a number of ways, *e.g.*, mg/mL or IU of the viral vector, mg or IU per kg or lb body weight of the subject, or in terms of the amount of antigenic polypeptide produced by the viral vector. The dose will vary depending on a number of factors, including frequency of administration; size and tolerance of the individual; severity of the H1N1 outbreak; risk of infection; and the route of administration. One of skill can determine how to modify a minimal dose depending on the above factors. The initial dose can be used as a baseline, and modified based on the reaction of the individual.

A "dosage form" refers to the particular format of the pharmaceutical, and depends on the route of administration. For example, a dosage form can be in a tablet or liquid, *e.g.,* for oral delivery, a suppository, *e.g.,* for rectal or vaginal administration, or a liquid for injection. A "single dosage form" refers both to the format of the pharmaceutical composition and the amount of active ingredient as discussed above.

"Protection" or "prevention" of infection refers to reducing the potential that an individual will develop H1N1 infection, *e.g.,* detected by observing symptoms, or by detecting viral or antibody titer. The term includes protection from further infection or worsening infection, *e.g.,* in the case of an individual that is already suffering from an infection, *e.g.,* from a different strain of H1N1 or a different virus. The potential for an infection can be reduced to such an extent that the individual does not display symptoms upon exposure to the infectious agent. For example, protecting an individual infection can refer to the ability of a chimeric viral vector of the present invention to prevent or reduce infection from occurring or recurring.

"Treating" or "reducing" H1N1 infection refers to reducing the amount of the infective agent (*e.g.,* number of cells or viral particles) in the individual, reducing the severity of symptoms, and/ or reducing the frequency of symptoms. In some embodiments, the number of infective agents is reduced by at least 10%, as compared, *e.g.,* to the individual before administration or to a control individual not undergoing treatment. In some embodiments the number of infective agents is reduced by at least 25%, 50%, 75%, 80%, or 90%. In some embodiments, the infection is no longer detectable, *e.g.*, by symptoms or general diagnostic techniques.

One of skill will appreciate that the terms protecting, preventing, and treatment are not absolute terms, but a general improvement of outcome compared to untreated or differently treated controls. For example, the compositions of the invention are considered protective if a treated individual or population is less likely to develop H1N1 infection than an individual or population that is not treated according to the invention, but exposed to similar conditions.

The term "infection" for the purposes of the invention refers to the presence of a potentially pathogenic H1N1 influenza strain in an individual. Infection does not necessarily imply that an infected individual will experience symptoms from the infective agent. H1N1 infection typically occurs via mucosal tissues, *e.g.,* epithelial linings of the reproductive tract, gastrointestinal tract, oral and esophageal surfaces, nasal cavity, and bronchial lining.

An agent "interferes with" or "protects from" infection when it reduces the ability of a targeted H1N1 strain to colonize the mucosal surface or penetrate the mucosal barrier, *e.g.,* to infect cells of the individual. For example, an agent interferes with infection if fewer cells are exposed to or infected by the virus. Reduction can be determined by comparison, *e.g.,* to the individual before treatment or to a control individual exposed to similar conditions but not undergoing treatment.

The term "isolated" is not an absolute term, but refers to a material that is substantially free of contaminants or components that accompany the material in its native state.

"Humoral immune responses" are mediated by cell free components of the blood, *i.e.*, plasma or serum, *e*.*g*., antibodies. Transfer of the serum or plasma from one individual to another transfers humoral immunity.

"Cell mediated immune responses" are mediated by antigen specific lymphocytes, *e.g.,* cytotoxic T cells (CTLs). Transfer of the antigen specific lymphocytes from one individual to another transfers immunity.

A "therapeutic dose" or "therapeutically effective amount" or "effective amount" of a chimeric viral vector or a composition comprising a chimeric viral vector is an amount of the vector or composition comprising the vector which prevents, alleviates, abates, or reduces the severity of symptoms of the targeted H1N1 virus.

Antibody refers to a polypeptide encoded by an immunoglobulin gene or fragments thereof that specifically binds and recognizes an antigen. The recognized immunoglobulin genes include the kappa, lambda, alpha, gamma, delta, epsilon, and mu constant region genes, as well as the myriad immunoglobulin variable region genes. Light chains are classified as either kappa or lambda. Heavy chains are classified as gamma, mu, alpha, delta, or epsilon, which in turn define the immunoglobulin classes, IgG, IgM, IgA, IgD and IgE, respectively.

T cells- refer to a particular class of lymphocytes that express a specific receptor (T cell receptor) encoded by a family of genes. The recognized T cell receptor genes include alpha, beta, delta, and gamma loci, and the T cell receptors typically (but not universally) recognize a combination of MHC plus a short peptide.

An adaptive immune response refers to specific recognition of an antigen by T cells (through the T cell receptor (TCR)) or B cells (through the BCR or antibody/ immunoglobulin).

Antigen presenting cells (APCs) present immunogenic peptides or fragments thereof to T cells to activate or enhance an immune response. APCs include dendritic cells, macrophages, B cells, monocytes and other cells that may be engineered to be efficient APCs. Such cells may, but need not, be genetically modified to increase the capacity for presenting the antigen, to improve activation and/or maintenance of the T cell response, to have anti-tumor effects per se and/or to be immunologically compatible with the receiver *(i.e.,* matched HLA haplotype). APCs may be isolated from any of a variety of biological fluids and organs including bone marrow, peripheral blood, tumor and peritumoral tissues, and may be autologous, allogeneic, syngeneic or xenogeneic cells. APCs typically utilize a receptor from the major histocompatability (MHC) locus to present short polypeptides to T cells.

Adjuvants are known in the art, and refer to non-specific immune response enhancers. Suitable adjuvants include, *e.g.,* dsRNA and dsRNA mimetics, cholera toxin, monophosphoryl lipid A (MPL), Freund's Complete Adjuvant, Freund's Incomplete Adjuvant, Quil A, and Al(OH). Adjuvants can also be those substances that cause APC activation and enhanced presentation of T cells through secondary signaling molecules like Toll-like receptors. Examples of Toll-like receptors include the receptors that recognize double-stranded RNA, bacterial flagella, LPS, CpG DNA, and bacterial lipopeptide (reviewed in Abreu et al., J Immunol, 174(8), 4453-4460 (2005)).

The terms "polypeptide," "peptide" and "protein" refer to a polymer of amino acid residues. The terms apply to amino acid polymers in which one or more amino acid residue is an artificial chemical mimetic of a corresponding naturally occurring amino acid, as well as to naturally occurring amino acid polymers and non-naturally occurring amino acid polymer.

The term "amino acid" refers to naturally occurring and synthetic amino acids, as well as amino acid analogs and amino acid mimetics that function in a manner similar to the naturally occurring amino acids. Naturally occurring amino acids are those encoded by the genetic code, as well as those amino acids that are later modified, *e.g.,* hydroxyproline, γ-carboxyglutamate, and O-phosphoserine. Amino acid analogs refers to compounds that have the same basic chemical structure as a naturally occurring amino acid, *i.e.,* a carbon that is bound to a hydrogen, a carboxyl group, an amino group, and an R group, *e.g.,* homoserine, norleucine, methionine sulfoxide, methionine methyl sulfonium. Such analogs have modified R groups (*e.g.,* norleucine) or modified peptide backbones, but retain the same basic chemical structure as a naturally occurring amino acid. Amino acid mimetics refers to chemical compounds that have a structure that is different from the general chemical structure of an amino acid, but that functions in a manner similar to a naturally occurring amino acid.

Amino acids may be referred to herein by either their commonly known three letter symbols or by the one-letter symbols recommended by the IUPAC-IUB Biochemical

Nomenclature Commission. Nucleotides, likewise, may be referred to by their commonly accepted single-letter codes.

"Conservatively modified variants" applies to both amino acid and nucleic acid sequences. With respect to particular nucleic acid sequences, conservatively modified variants refers to those nucleic acids which encode identical or essentially identical amino acid sequences, or where the nucleic acid does not encode an amino acid sequence, to essentially identical sequences. Because of the degeneracy of the genetic code, a large number of functionally identical nucleic acids encode any given protein. For instance, the codons GCA, GCC, GCG and GCU all encode the amino acid alanine. Thus, at every position where an alanine is specified by a codon, the codon can be altered to any of the corresponding codons described without altering the encoded polypeptide. Such nucleic acid variations are "silent variations," which are one species of conservatively modified variations. Every nucleic acid sequence herein which encodes a polypeptide also describes every possible silent variation of the nucleic acid. One of skill will recognize that each codon in a nucleic acid (except AUG, which is ordinarily the only codon for methionine, and TGG, which is ordinarily the only codon for tryptophan) can be modified to yield a functionally identical molecule. Accordingly, each silent variation of a nucleic acid which encodes a polypeptide is implicit in each described sequence.

As to amino acid sequences, one of skill will recognize that individual substitutions, deletions or additions to a nucleic acid, peptide, polypeptide, or protein sequence which alters, adds or deletes a single amino acid or a small percentage of amino acids in the encoded sequence is a "conservatively modified variant" where the alteration results in the substitution of an amino acid with a chemically similar amino acid. Conservative substitution tables providing functionally similar amino acids are well known in the art. Such conservatively modified variants are in addition to and do not exclude polymorphic variants, interspecies homologs, and alleles of the invention.

The following eight groups each contain amino acids that are conservative substitutions for one another:
1) Alanine (A), Glycine (G);
2) Aspartic acid (D), Glutamic acid (E);
3) Asparagine (N), Glutamine (Q);
4) Arginine I, Lysine (K);
5) Isoleucine (I), Leucine (L), Methionine (M), Valine (V);
6) Phenylalanine (F), Tyrosine (Y), Tryptophan (W);
7) Serine (S), Threonine (T); and
8) Cysteine (C), Methionine (M)
(*see, e.g.*, Creighton, Proteins (1984)).

The phrase "selectively (or specifically) hybridizes to" refers to the binding, duplexing, or hybridizing of a polynucleotide only to a particular nucleotide sequence, *i.e.,* according to Watson-Crick complementarity.

Nucleic acids that do not hybridize to each other under given conditions are still considered substantially identical if the polypeptides which they encode are substantially identical. This occurs, for example, when a copy of a nucleic acid is created using the maximum codon degeneracy permitted by the genetic code. In such cases, the nucleic acids typically hybridize under moderately stringent hybridization conditions. Exemplary "moderately stringent hybridization conditions" include a hybridization in a buffer of 40% formamide, 1 M NaCl, 1% SDS at 37°C, and a wash in 1X SSC at 45°C. A positive hybridization is at least twice background. Those of ordinary skill will readily recognize that alternative hybridization and wash conditions can be utilized to provide conditions of similar stringency.

The phrase "specifically (or selectively) binds" to an antibody or "specifically (or selectively) immunoreactive with," when referring to a protein or peptide, refers to a binding reaction that is determinative of the presence of the protein in a heterogeneous population of proteins and other biologics. Thus, under designated immunoassay conditions, the specified antibodies bind to a particular protein at least two times the background and do not substantially bind in a significant amount to other proteins present in the sample. Specific binding to an antibody under such conditions may require an antibody that is selected for its specificity for a particular protein. For example, polyclonal antibodies raised to fusion proteins can be selected to obtain only those polyclonal antibodies that are specifically immunoreactive with fusion protein and not with individual components of the fusion proteins. This selection may be achieved by subtracting out antibodies that cross-react with the individual antigens.

A variety of immunoassay formats may be used to select antibodies specifically immunoreactive with a particular protein. For example, solid-phase ELISA immunoassays are routinely used to select antibodies specifically immunoreactive with a protein (*see*, *e.g.*, Harlow & Lane, Antibodies, A Laboratory Manual (1988), for a description of immunoassay formats and conditions that can be used to determine specific immunoreactivity). Typically a specific or selective reaction will be at least twice background signal or noise and more typically more than 10 to 100 times background.

The terms "identical" or percent "identity," in the context of two or more nucleic acids *(e.g.,* a dsRNA or antigen encoding sequence) or polypeptide sequences, refer to two or more sequences or subsequences that are the same or have a specified percentage of amino acid residues or nucleotides that are the same *(i.e.,* 50%, 55%, 60%, 65%, 70%, 75%, 80%, 85%, 90%, 91%, 92%, 93%, 94%, 95%, 96%, 97%, 98%, 99%, or more identity over a specified region), when compared and aligned for maximum correspondence over a comparison window, or designated region as measured using one of the following sequence comparison algorithms or by manual alignment and visual inspection. Such sequences are then said to be "substantially identical." This definition also refers to the compliment of a test sequence. Optionally, the identity exists over a region that is at least about 10 to about 100, about 20 to about 75, about 30 to about 50 amino acids or nucleotides in length.

For sequence comparison, typically one sequence acts as a reference sequence, to which test sequences are compared. When using a sequence comparison algorithm, test and reference sequences are entered into a computer, subsequence coordinates are designated, if necessary, and sequence algorithm program parameters are designated. Default program parameters can be used, or alternative parameters can be designated. The sequence comparison algorithm then calculates the percent sequence identities for the test sequences relative to the reference sequence, based on the program parameters.

A "comparison window", as used herein, includes reference to a segment of any one of the number of contiguous positions from about 10 to about 500, about 25 to about 200, 50 to about 150, in which a sequence may be compared to a reference sequence of the same number of contiguous positions after the two sequences are optimally aligned. Methods of alignment of sequences for comparison are well-known in the art. Optimal alignment of sequences for comparison can be conducted, *e.g.,* by the local homology algorithm of Smith & Waterman, Adv. Appl. Math. 2:482 (1981), by the homology alignment algorithm of Needleman & Wunsch, J. Mol. Biol. 48:443 (1970), by the search for similarity method of Pearson & Lipman, Proc. Nat'l. Acad. Sci. USA 85:2444 (1988), by computerized implementations of these algorithms (GAP, BESTFIT, FASTA, and TFASTA in the Wisconsin Genetics Software Package, Genetics Computer Group, 575 Science Dr., Madison, WI), or by manual alignment and visual inspection (*see, e.g.,* Current Protocols in Molecular Biology (Ausubel et al., eds. 1995 supplement)).

An example of an algorithm that is suitable for determining percent sequence identity and sequence similarity are the BLAST and BLAST 2.0 algorithms, which are described in Altschul et al., Nuc. Acids Res. 25:3389-3402 (1977) and Altschul et al., J. Mol. Biol. 215:403-. 410 (1990), respectively. Software for performing BLAST analyses is publicly available through the National Center for Biotechnology Information (at the website for NCBI, ncbi.nlm.nih.gov). This algorithm involves first identifying high scoring sequence pairs (HSPs) by identifying short words of length W in the query sequence, which either match or satisfy some positive-valued threshold score T when aligned with a word of the same length in a database sequence. T is referred to as the neighborhood word score threshold (Altschul *et al., supra*). These initial neighborhood word hits act as seeds for initiating searches to find longer HSPs containing them. The word hits are extended in both directions along each sequence for as far as the cumulative alignment score can be increased. Cumulative scores are calculated using, for nucleotide sequences, the parameters M (reward score for a pair of matching residues; always > 0) and N (penalty score for mismatching residues; always < 0). For amino acid sequences, a scoring matrix is used to calculate the cumulative score. Extension of the word hits in each direction are halted when: the cumulative alignment score falls off by the quantity X from its maximum achieved value; the cumulative score goes to zero or below, due to the accumulation of one or more negative-scoring residue alignments; or the end of either sequence is reached. The BLAST algorithm parameters W, T, and X determine the sensitivity and speed of the alignment. The BLASTN program (for nucleotide sequences) uses as defaults a wordlength (W) of 11, an expectation (E) or 10, M=5, N=-4 and a comparison of both strands. For amino acid sequences, the BLASTP program uses as defaults a wordlength of 3, and expectation (E) of 10, and the BLOSUM62 scoring matrix (*see* Henikoff & Henikoff, Proc. Natl. Acad. Sci. USA 89:10915 (1989)) alignments (B) of 50, expectation (E) of 10, M=5, N=-4, and a comparison of both strands.

### III. Compositions disclosed

The disclosure provides compositions comprising chimeric viral vectors. In some aspects of the disclosure described herein, the chimeric viral vectors comprise a first promoter operably linked to a nucleic acid encoding an antigenic H1N1 polypeptide and a second promoter operably linked to a nucleic acid encoding a TLR3 agonist. The first and second promoters may be the same or different, inducible, constitutive, or tissue-specific. In some aspects of the disclosure described herein, the first and second promoters are independently selected from: the beta actin promoter and the CMV promoter.

In some aspects of the disclosure, the chimeric viral vector comprises an adenoviral genome (minus the E1 and E3 genes) and a nucleic acid encoding a dsRNA. The chimeric vector can be administered to a cell that expresses the Ad E1 gene such that recombinant adenovirus (rAd) is produced by the cell. This rAd can be harvested and is capable of a single round of infection that will deliver the transgenic composition to another cell within a mammal in order to elicit immune responses to the antigenic polypeptide.

### A. Suitable Viral Vectors

Any viral expression vector can be used for the present disclosure. Viral vaccines are typically attenuated *(e.g.,* replication incompetent). Suitable viral expression vectors described herein can be derived from adenoviruses, modified vaccinia ankara (MVA; *see, e.g.,* Kumar and Seth (2004) Gene Ther. Mol. Biol. 8:193-200), vaccinia viruses, herpes viruses, baculoviruses, adeno-associated viruses (AAV), and rubella virus (*e.g.,* as described in Pugachev et al. (2000) J. Virology 74:10811-15). In the invention, the vector is an adenoviral vector, *e.g.,* derived from adenovirus 5, for example, Ad5 with deletions of the E1/E3 regions and Ad5 with a deletion of the E4 region. Other suitable adenoviral vectors include strains 2, orally tested strains 4 and 7, enteric adenoviruses 40 and 41, and other strains (*e.g.* Ad34) that are sufficient for delivering an antigen and eliciting an adaptive immune response to the transgene antigen [Lubeck et al., Proc Natl Acad Sci U S A, 86(17), 6763-6767 (1989); Shen et al., J Virol, 75(9), 4297-4307 (2001); Bailey et al., Virology, 202(2), 695-706 (1994)]. In some embodiments, the adenoviral vector is a live, replication incompetent adenoviral vector (such as E1 and E3 deleted rAd5), live and attenuated adenoviral vector (such as the E1B55K deletion viruses), or a live adenoviral vector with wild-type replication.

The transcriptional and translational control sequences in expression vectors to be used in transforming vertebrate cells *in vivo* may be provided by viral sources. For example, commonly used promoters and enhancers are derived, *e.g.,* from beta actin, adenovirus, simian virus (SV40), and human cytomegalovirus (CMV). For example, vectors allowing expression of proteins under the direction of the CMV promoter, SV40 early promoter, SV40 later promoter, metallothionein promoter, murine mammary tumor virus promoter, Rous sarcoma virus promoter, transducer promoter, or other promoters shown effective for expression in mammalian cells are suitable. Further viral genomic promoters, control and/or signal sequences can be used, provided such control sequences are compatible with the host cell chosen.

### B. H1N1 Polypeptides

Nucleic acids encoding suitable heterologous polypeptides described herein may be derived from H1N1 influenza antigens. H1N1 infects mucosal tissues, such as intestine, thus antigens that give rise to a robust mucosal response are of particular interest. Antigenic polypeptides described herein include H1N1 viral surface antigens and fragments thereof. Antigenic polypeptides described herein include, *e.g.,* HA, NA, MP, and NP from H1N1 influenza.

One of skill in the art will understand how to identify and isolate antigenic polypeptides from a newly isolated or particularly virulent H1N1 strain, *e.g.,* using comparisons to known influenza sequences.

A recombinant expression vector described herein can include nucleotide sequences encoding an immunogenic polypeptide operably linked to suitable transcriptional or translational regulatory elements derived from mammalian or viral genes. Such regulatory elements include promoters, a sequence encoding suitable mRNA ribosomal binding sites, and sequences which control the termination of transcription and translation. An origin of replication, a selectable marker to facilitate recognition of transformants, and/or an operator sequence to control transcription may additionally be incorporated. In some aspects of the disclosure described herein, the components of the recombinant expression vector can be divided between more than one virus such that the gene products are on two different vectors, and the vectors are used for co-transduction to provide all the gene products in trans. There may be reasons to divide up the gene products such as size limitations for insertions, or toxicity of the combined gene products to the virus produce cell-lines.

### C. TLR-3 Agonists

TLR-3 agonists are used to enhance the immune response to the H1N1 polypeptide. The TLR-3 agonist can be delivered simultaneously on the same expression vector encoding an antigen of interest. In some aspects of the disclosure described herein, the TLR-3 agonist can be delivered separately *(i.e.,* temporally or spatially) from the expression vector encoding an antigen of interest. For example, the expression vector may be administered via a non-parenteral route (*e.g.,* orally, intranasally, or mucosally), while the TLR-agonist is delivered by a parenteral route (*e.g.,* intramuscularly, intraperitoneally, or subcutaneously).

TLR-3 agonists include, for example, short hairpin RNA, virally derived RNA, short segments of RNA that can form double-strands, and short interfering RNA (siRNA). In some embodiments of the invention, the TLR-3 agonist is virally derived dsRNA, such as for example, a dsRNA derived from a Sindbis virus or dsRNA viral intermediates [Alexopoulou et al, Nature 413:732-8 (2001)].

In a preferred embodiment of the invention, the TLR-3 agonist is a short hairpin RNA. Short hairpin RNA sequences typically comprise two complementary sequences joined by a linker sequence. The particular linker sequence is not a critical aspect of the invention. Any appropriate linker sequence can be used so long as it does not interfere with the hybridization of the two complementary sequences to form a dsRNA. Similarly, the sequence of the complementary stretches is not necessarily critical, as long as the strands form a double-stranded molecule. The strands need not be 100% complementary as long as the double-stranded structure is maintained. The complementary stretch is typically in the range of 10-30 nucleotides, *e.g.,* 4-10, 5-15, 5-20, 10-25, 20-30, or 10-30 nucleotides, but can be longer, *e.g.,* 20-100 nucleotides in length.

In some embodiments, the sequence encoding the short hairpin RNA comprises a sequence set forth in any one of SEQ ID NOs:1-6, a sequence with substantial identity to a sequence set forth in SEQ ID NOs:1-6, or a variant of a sequence set forth in SEQ ID NOs:1-6. The dsRNA that is a TLR-3 agonist does not encode a particular polypeptide, but results in production of a pro-inflammatory cytokine (*e.g.* IL-6, IL-8, TNF-alpha, IFN-alpha, IFN-beta) when contacted with a responder cell (*e.g.,* a dendritic cell, a peripheral blood mononuclear cell, or a macrophage) *in vitro* or *in-vivo*.

The nucleic acid encoding the TLR-3 agonist (*e.g.,* an expressed dsRNA) and the chimeric viral vector comprising the nucleic acid encoding an H1N1 antigen are administered in the same formulation. In other cases described herein, the nucleic acid encoding the TLR-3 agonist and the chimeric viral vector comprising the nucleic acid encoding the H1N1 antigen are administered in different formulations, either simultaneously or sequentially.

In some embodiments, the nucleic acid encoding the TLR-3 agonist and the nucleic acid encoding the H1N1 antigen are under the control of the same promoter. In other embodiments, the nucleic acid encoding the TLR-3 agonist and the nucleic acid encoding the H1N1 antigen are under the control of different promoters.

Several chemically synthesized analogs to double-stranded RNA are commercially available. These include polyinosine-polycytidylic acid (poly I:C), polyadenylic:polyuridylic acid (poly A:U), and poly I:poly C. Antibodies (or cross-linking of antibodies) to TLR-3 can also lead to IFN- beta or pro-inflammatory cytokine production [Matsumoto et al, Biochem. Biophys. Res. Commun. 24:1364 (2002), de Bouteiller et al, J Biol. Chem. 18:38133-45 (2005)]. Commercially available siRNA segments of any sequence can also be obtained through sources such as Invitrogen.

### IV. Pharmaceutical Compositions

Pharmaceutical compositions comprising the vectors described herein can include pharmaceutically acceptable carriers, which may be biologically active or inactive. Pharmaceutical compositions can generally be used for prophylactic and therapeutic purposes. In some embodiments, the pharmaceutical composition can be designed to protect against stomach degradation. For the oral environment, several such compositions are available including the Eudragit and the TimeClock release systems as well as other methods specifically designed for adenovirus [Lubeck et al., Proc Natl Acad Sci USA, 86(17), 6763-6767 (1989); Chourasia and Jain, J Pharm Pharm Sci, 6(1), 33-66 (2003)]. There are also several methods already described for microencapsulation of DNA and drugs for oral delivery (*see, e.g.*, U.S. Patent Publication No. 2004043952). In some embodiments, the Eudragit system can be used to deliver the chimeric viral vector to the lower small intestine. However, delivery to other locations of the small intestine is also advantageous for protection against H1N1.

The chimeric viral vectors of the invention can be delivered using any delivery systems known in the art. Numerous gene delivery techniques are well known, such as those described by Rolland (1998) Crit. Rev. Therap. Drug Carrier Systems 15:143-198, and references cited therein.

It will be apparent that an immunogenic composition can contain pharmaceutically acceptable salts of the polynucleotides encoding the H1N1 immunogenic polypeptides. Such salts may be prepared from pharmaceutically acceptable non-toxic bases, including organic bases (*e.g.,* salts of primary, secondary and tertiary amines and basic amino acids) and inorganic bases (*e.g.,* sodium, potassium, lithium, ammonium, calcium and magnesium salts). Some particular examples of salts include phosphate buffered saline and saline for injection.

Any suitable carrier known to those of ordinary skill in the art may be employed in the pharmaceutical compositions of this invention. Suitable carriers include, for example, water, saline, alcohol, a fat, a wax, a buffer, a solid carrier, such as mannitol, lactose, starch, magnesium stearate, sodium saccharine, talcum, cellulose, glucose, sucrose, and magnesium carbonate, or biodegradable microspheres (*e.g.,* polylactate polyglycolate). Suitable biodegradable microspheres are disclosed, for example, in US Patent Nos. 4,897,268; 5,075,109; 5,928,647; 5,811,128; 5,820,883. The chimeric viral vector can be encapsulated within the biodegradable microsphere or associated with the surface of the microsphere.

Such compositions can comprise buffers (*e.g.,* neutral buffered saline or phosphate buffered saline), carbohydrates (*e.g.,* glucose, mannose, sucrose or dextrans), mannitol, proteins, polypeptides or amino acids such as glycine, antioxidants, bacteriostats, chelating agents such as EDTA or glutathione, adjuvants (*e.g.*, aluminum hydroxide), solutes that render the formulation isotonic, hypotonic or weakly hypertonic with the blood of a recipient, suspending agents, thickening agents and/or preservatives. Alternatively, compositions of the present invention can be formulated as a lyophilizate. Compounds can also be encapsulated within liposomes using well known technology.

In some embodiments, the compositions can comprise an additional adjuvant. Suitable adjuvants include, for example, the lipids and non-lipid compounds, cholera toxin (CT), CT subunit B, CT derivative CTK63, E. coli heat labile enterotoxin (LT), LT derivative LTK63, Al(OH)₃, and polyionic organic acids as described in *e.g.,* WO 04/020592, Anderson and Crowle, Infect. Immun. 31(1):413-418 (1981), Roterman et al., J. Physiol. Pharmacol., 44(3):213-32 (1993), Arora and Crowle, J. Reticuloendothel. 24(3):271-86 (1978), and Crowle and May, Infect. Immun. 38(3):932-7 (1982)). Suitable polyionic organic acids include for example, 6,6'-[3,3'-demithyl[1,1'-biphenyl]-4,4'-diyl]bis(azo)bis[4-amino-5-hydroxy-1,3-naphthalene-disulfonic acid] (Evans Blue) and 3,3'-[1,1'biphenyl]-4,4'-diylbis(azo)bis[4-amino-1-naphthalenesulfonic acid] (Congo Red). It will be appreciated by those of skill in the art that the polyionic organic acids can be used for gene-based vaccination methods in conjunction with any route of administration.

Other suitable adjuvants include topical immunomodulators such as members of the imidazoquinoline family, *e.g.,* imiquimod and resiquimod (see, *e.g.,* Hengge et al., Lancet Infect. Dis. 1(3):189-98 (2001).

Additional suitable adjuvants are commercially available as, for example, additional alum-based adjuvants (*e.g.,* Alhydrogel, Rehydragel, aluminum phosphate, Algammulin); oil based adjuvants (Freund's Incomplete Adjuvant and Complete Adjuvant (Difco Laboratories, Detroit, Mich.), Specol, RIBI, TiterMax, Montanide ISA50 or Seppic MONTANIDE ISA 720); nonionic block copolymer-based adjuvants, cytokines (*e.g.,* GM-CSF or Flat3-ligand); Merck Adjuvant 65 (Merck and Company, Inc., Rahway, N.J.); AS-2 (SmithKline Beecham, Philadelphia, Pa.); salts of calcium, iron or zinc; an insoluble suspension of acylated tyrosine; acylated sugars; cationically or anionically derivatized polysaccharides; polyphosphazenes; biodegradable microspheres; monophosphoryl lipid A and Quil A. Cytokines, such as GM-CSF or interleukin-2, -7, or -12, are also suitable adjuvants. Hemocyanins (*e.g.,* keyhole limpet hemocyanin) and hemoerythrins can also be used. Polysaccharide adjuvants such as, for example, chitin, chitosan, and deacetylated chitin are also suitable as adjuvants. Other suitable adjuvants include muramyl dipeptide (MDP, N acetylmuramyl L alanyl D isoglutamine) bacterial peptidoglycans and their derivatives (*e.g.,* threonyl-MDP, and MTPPE). BCG and BCG cell wall skeleton (CWS) can also be used as adjuvants, with or without trehalose dimycolate. Trehalose dimycolate can also be used (see, *e.g.,* U.S. Pat. No. 4,579,945). Detoxified endotoxins are also useful as adjuvants alone or in combination with other adjuvants (see, *e.g.,* U.S. Pat. Nos. 4,866,034; 4,435,386; 4,505,899; 4,436,727; 4,436,728; 4,505,900; and 4,520,019. The saponins QS21, QS17, QS7 are also useful as adjuvants (see, *e.g.,* U.S. Pat. No. 5,057,540; EP 0362 279; WO 96/33739; and WO 96/11711). Other suitable adjuvants include Montanide ISA 720 (Seppic, France), SAF (Chiron, Calif., United States), ISCOMS (CSL), MF-59 (Chiron), the SBAS series of adjuvants (*e.g.,* SBAS-2, SBAS-4 or SBAS-6 or variants thereof, available from SmithKline Beecham, Rixensart, Belgium), Detox (Corixa, Hamilton, Mont.), and RC-529 (Corixa, Hamilton, Mont.).

Superantigens are also contemplated for use as adjuvants. Superantigens include Staphylococcus exoproteins, such as the α, β, γ and Δ enterotoxins from *S. aureus* and *S*. *epidermidis,* and the α, β, γ and Δ E. coli exotoxins. Common Staphylococcus enterotoxins are known as staphylococcal enterotoxin A (SEA) and staphylococcal enterotoxin B (SEB), with enterotoxins through E (SEE) being described (Rott *et al.,* 1992). *Streptococcus pyogenes B* (SEB), *Clostridium perfringens* enterotoxin (Bowness *et al.,* 1992), cytoplasmic membrane-associated protein (CAP) from *S. pyogenes* (Sato *et al.,* 1994) and toxic shock syndrome toxin 1 (TSST 1) from *S. aureus* (Schwab *et al.*, 1993) are further useful superantigens.

Within the pharmaceutical compositions provided herein, the adjuvant composition can be designed to induce, *e.g.,* an immune response predominantly of the Th1 or Th2 type. High levels of Th1-type cytokines (*e.g.,* IFN-gamma, TNF-alpha, IL-2 and IL-12) tend to favor the induction of cell mediated immune responses to an administered antigen. In contrast, high levels of Th2-type cytokines (*e.g.,* IL-4, IL-5, IL-6 and IL-10) tend to favor the induction of humoral immune responses. Oral delivery of a composition comprising an immunogenic H1N1 polypeptide can induce an immune response that includes Thl- and Th2-type responses.

The compositions described herein may be administered as part of a sustained release formulation (*i.e.,* a formulation such as a capsule or sponge that effects a slow release of compound following administration). Such formulations may generally be prepared using well known technology (*see, e.g.,* Coombes et al. (1996) Vaccine 14:1429-1438). Sustained-release formulations may contain a polypeptide, polynucleotide or antibody dispersed in a carrier matrix and/or contained within a reservoir surrounded by a rate controlling membrane.

Carriers for use within such formulations are biocompatible, and may also be biodegradable; preferably the formulation provides a relatively constant level of active component release. Such carriers include microparticles of poly(lactide-co-glycolide), as well as polyacrylate, latex, starch, cellulose and dextran. Other delayed-release carriers include supramolecular biovectors, which comprise a non-liquid hydrophilic core *(e.g.,* a cross-linked polysaccharide or oligosaccharide) and, optionally, an external layer comprising an amphiphilic compound (see, *e.g.,* WO 94/20078; WO 94/23701; and WO 96/06638). The amount of active compound contained within a sustained release formulation depends upon the site of implantation, the rate and expected duration of release and the nature of the condition to be treated or prevented.

The pharmaceutical composition can be presented in unit-dose or multi-dose containers, such as sealed ampoules or vials. Such containers are preferably hermetically sealed to preserve sterility of the formulation until use. In general, formulations can be stored as suspensions, solutions or emulsions in oily or aqueous vehicles. Alternatively, a pharmaceutical composition may be stored in a freeze-dried condition requiring only the addition of a sterile liquid carrier immediately prior to use.

### V. Therapeutic Uses of the Invention

The invention provides vaccines to reduce the likelihood or severity of H1N1 infection. As used herein, a "subject" or a "patient" refers to any warm-blooded animal, such as, for example, a rodent, a feline, a canine, or a primate, *e.g.,* human.

The immunogenic compositions may be used to treat at any stage, *e.g.,* to prevent infection with a different strain of H1N1 in an individual that has already experienced infection with an H1N1 strain. Within such methods, pharmaceutical compositions are typically administered to a patient. The patient may or may not yet be infected or exposed. Accordingly, the above pharmaceutical compositions may be used to prevent the development of infection or symptoms. Infection or exposure can be diagnosed according to methods known in the art, *e.g.,* by measuring viral titer or viral-specific antibody in a sample from the patient.

Immunotherapy is typically active immunotherapy, in which treatment relies on the *in vivo* stimulation of the endogenous host immune system to react against virus or virally infected cells.

Frequency of administration of the prophylactic or therapeutic compositions described herein, as well as dosage, will vary from individual to individual, and may be readily established using standard techniques. For example, between 1 and 10 doses can be administered over a set period of time (*e.g.,* 1 or 2 weeks, or 1, 2, or 3 months) or over the course of a pandemic outbreak. In some embodiments, 3 doses are administered, at intervals of 1 month. In some embodiments, 2-3 doses are administered every 2-3 months. Booster vaccinations can be given periodically thereafter, *e.g.,* for the duration of the pandemic, or if there is a risk of reemergence of the targeted strain or a similar strain.

One of skill in medicine will appreciate that dosage and frequency of administration will vary for individual patients. A suitable dose is an amount of the composition that, when administered as described above, is capable of promoting an anti-viral immune response that is at least 10-50% above the basal (*i.e*., untreated) level. Such response can be monitored by measuring the specific antibodies in a patient or by vaccine-dependent generation of cytolytic T cells capable of killing virally infected cells *in vitro.* Such vaccines should also be capable of causing an immune response that leads to an improved clinical outcome in vaccinated patients as compared to non-vaccinated patients.

In general, an appropriate dosage and treatment regimen provides the active compound(s) in an amount sufficient to provide therapeutic and/or prophylactic benefit. Such a response can be monitored by establishing an improved clinical outcome (*e.g.,* reduced severity of symptoms such as weight loss, no or low detectable viral titer, greater chance of survival) in treated patients as compared to non-treated patients. Such immune responses can generally be evaluated using standard proliferation, cytotoxicity or cytokine assays described above. Immune responses can be determined using samples obtained from a patient before and after treatment, or compared to an averaged response of a population, *e.g.,* of similar individuals.

Detection of immunocomplexes formed between immunogenic polypeptides and antibodies in body fluid can be used to monitor the effectiveness of therapy. Samples of body fluid taken from an individual prior to and subsequent to vaccination can be analyzed for the immunocomplexes by the methodologies described above. A substantial change in the number of immunocomplexes in the second sample (post-vaccination) relative to the first sample (pre-vaccination) can reflect successful vaccination.

### A. Administration of the Compositions of the Invention

A composition comprising the chimeric viral vector described herein can be administered by any non-parenteral route (*e.g.,* orally, intranasally, or mucosally via, for example, the vagina, lungs, salivary glands, nasal cavities, small intestine, colon, rectum, tonsils, or Peyer's patches). The composition can be administered alone or with an additional adjuvant as described above.

### B. Detection of an Immune Response to Antigens of Interest

An immune response to the H1N1 antigenic polypeptide can be detected using any means know in the art including, for example detecting specific activation of CD4⁺ or CD8⁺ T cells or by detecting the presence of antibodies that specifically bind to the polypeptide.

Specific activation of CD4⁺ or CD8⁺ T cells associated with a mucosal, humoral, or cell-mediated immune response may be detected in a variety of ways. Methods for detecting specific T cell activation include, but are not limited to, detecting the proliferation of T cells, the production of cytokines (*e.g.,* lymphokines), or the generation of cytolytic activity (*i.e.*, generation of cytotoxic T cells specific for the immunogenic polypeptide). For CD4⁺ T cells, activation can be determined by detecting proliferation. For CD8⁺ T cells, activation can be detected by the generation of cytolytic activity using ⁵¹Cr release assays (see, *e.g.,* Brossart and Bevan, Blood 90(4): 1594-1599 (1997) and Lenz et al., J. Exp. Med. 192(8):1135-1142 (2000)).

Detection of the proliferation of T cells may be accomplished by a variety of known techniques. For example, T cell proliferation can be detected by measuring the rate of DNA synthesis. T cells which have been stimulated to proliferate exhibit an increased rate of DNA synthesis. A typical way to measure the rate of DNA synthesis is, for example, by pulse-labeling cultures of T cells with tritiated thymidine, a nucleoside precursor which is incorporated into newly synthesized DNA. The amount of tritiated thymidine incorporated can be determined using a liquid scintillation spectrophotometer. Other ways to detect T cell proliferation include measuring increases in interleukin-2 (IL-2) production, Ca2+ flux, or dye uptake, such as 3-(4,5-dimethylthiazol-2-yl)-2,5-diphenyltetrazolium. Alternatively, synthesis of lymphokines (*e.g.,* interferon-gamma) can be measured or the relative number of T cells that can respond to the immunogenic polypeptide may be quantified.

Antibody immune responses (humoral immune responses or B cell responses), including mucosal antibody responses can be detected using immunoassays known in the art [Tucker et al., Mol Therapy, 8, 392-399 (2003); Tucker et al., Vaccine, 22, 2500-2504 (2004)]. Suitable immunoassays include the double monoclonal antibody sandwich immunoassay technique of David *et al.* (U.S. Pat. No. 4,376,110); monoclonal-polyclonal antibody sandwich assays (Wide et al., in Kirkham and Hunter, eds., Radioimmunoassay Methods, E. and S. Livingstone, Edinburgh (1970)); the "western blot" method of Gordon *et al.* (U.S. Pat. No. 4,452,901); immunoprecipitation of labeled ligand (Brown et al. (1980) J. Biol. Chem. 255:4980-4983); enzyme-linked immunosorbent assays (ELISA) as described, for example, by Raines et al. (1982) J. Biol. Chem. 257:5154-5160; immunocytochemical techniques, including the use of fluorochromes (Brooks et al. (1980) Clin. Exp. Immunol. 39:477); and neutralization of activity (Bowen-Pope et al. (1984) Proc. Natl. Acad. Sci. USA 81:2396-2400). In addition to the immunoassays described above, a number of other immunoassays are available, including those described in U.S. Pat. Nos. 3,817,827; 3,850,752; 3,901,654; 3,935,074; 3,984,533; 3,996,345; 4,034,074; and 4,098,876.

The examples are provided to illustrate the invention but not to limit its scope. Other variants of the invention will be readily apparent to one of ordinary skill in the art and are encompassed by the appended claims.

### VI. Examples

### Example 1: Construction of a chimeric adenoviral vector (DX1) encoding a dsRNA adjuvant and an H1N1 HA polypeptide

The coding sequence for HA from influenza strain A/CA/04/09 (synthesized by Blue Heron) was cloned into a KpnI restriction site of a shuttle vector (pShuttleCMV, Qbiogene). The start codon for the HA sequence is immediately downstream of a CMV promoter with a small intron. A bGH poly A sequence immediately follows the HA gene. The pShuttle vector also includes a sequence encoding a dsRNA hairpin adjuvant (luc1, SEQ ID NO:1), so that both antigen and adjuvant are both encoded on the same vector but with separate CMV promoters. This shuttle vector is called pCMV-HA-CMV-1uc1.

Homologous recombination of pCMV-HA-CMV-1uc1 with the adenoviral backbone, pAd (Qbiogene), was performed in order to generate a plasmid construct capable of producing a recombinant Adenoviral vector (E1/E3 deleted) that expressed the HA transgene and the sequence coding for the dsRNA hairpin luc1. This vector is called pAd-DX1, and shown in SEQ ID NO:7. Recombinant Ad was generated by transfecting a linerized pAd-DX1 expression construct into HEK293 cells. This viral vector is called DX1. Viral titers (infectious units (IU/ ml) were measured by a hexon immunostaining assay which is similar to the QuickTiterTM Immunoassay described by Cell Biolabs Inc. (San Diego, CA)

### Example 2: Oral DX1 is superior to injected rAd5 for inducing an antigen specific immune responses in mucosal tissues

Generating a high antibody titer to an orally-administered, gene-based antigen delivery poses a challenge. We sought to determine whether the DX1 vector could induce substantial systemic and mucosal antibody titers. As explained above, DX1 expresses HA under control of the CMV promoter in an E1/E3 deleted adenovirus type 5 vector. Balb/c female mice were administered DX1 vector by oral gavage on weeks 0 and 5 with 1.0x10⁷ IU or 1.0x10⁸ IU DX1 (labeled Ad-CA04/09-Adj in Figure 1) or by intramuscular (i.m.) immunization of 1.0x10⁸ IU of DX1 (N=6 for each group). Control groups were vaccinated with a similar Ad vector expressing an H1N1 HA antigen from influenza lab strain A/PR/8/34 (labeled Ad-PR8/34-Adj in Figure 1). A/PR/8/34 is not considered highly similar to the A/CA/04/09 H1N1 strain. The percent amino acide identity of the HA protein is on the order of 75-85%. The control Ad vector was also administered either orally (labeled p.o.) or by i.m. injection. Untreated mice provided an additional control.

To determine systemic antibody response, we measured antibody titers in plasma post-vaccination. Antibody titers to A/CA/4/09 HA were measured in the plasma 2, 3, 5 and 8 weeks after the initial virus administration by anti-HA IgG ELISA (Figure 1A). Oral and i.m. immunizations with DX1 produced comparable systemic anti-HA IgG immunity by 8 weeks post vaccination.

Hemagglutinination inhibition (HAI) antibody titers were also measured in plasma at 3, 5, and 8 weeks post-vaccinataion (Figure 1B). Significant HAI titers were elicitated in both oral (p.o.) groups. By 8 weeks, the high oral dose showed essentially equivalent HAI titer as that elicited by muscle injection. The PR/8/34 HA vector did not induce HAI titers above the background of the assay, as the ELISA relied on antibody binding to the HA antigen from A/CA/4/09.

Mucosal IgA antibody titers to HA were measured in fecal samples at week 8 post-administration (Figure 1C), vaginal lavages at week 10 post-administration (Figure 1D), and lung lavages at week 12 post-administration (Figure 1E). These results demonstrate that oral delivery of DX1 vector performed significantly better than i.m. delivery in eliciting antibody responses to the protein HA in both local (intestine) and distal (vagina & lungs) mucosal tissues. In particular, the average antibody titer to HA was 17-fold better in intestinal tissues (fecal IgA) after oral immunizations than after i.m. vaccination (Figure 1C).

Surprisingly, a 10-fold lower dose of orally administered DX1 induced an equivalent or better specific IgA response than i.m. delivery of DX1 in lung washes, vaginal washes, and fecal pellets (Figures 1C, 1D, 1E). In addition, oral administration of the poorly cross-reactive PR/8/34 vector resulted in a specific IgA response similar to that of the i.m. administered DX1. The specific IgA results indicate that the oral use of DX1 provides superior protection against H1N1 influenza, a mucosally invading pathogen. Thus, use of the present adenoviral vaccine approach can allow for lower effective vaccine doses. In addition, vaccination with a mismatched antigen can still impart some specific immune protection. These are important considerations during a pandemic, when vaccine supply is critical and the availability of a completely matched vaccine strain may not be possible.

### Example 3: Demonstration that oral delivery of DX1 can protect animals against influenza challenge

Mice were immunized with the groups listed in Table 1 on weeks 0 and 4. Oral immunization was performed by oral gavage with either 1x10⁸ or 1x10⁷ IU of DX1 (Groups A and C). In order to control for delivery, an i.m. immunization with DX1 was also performed (Group B). To control for possible downstream effects of the adjuvant, we used an adjuvant control vector that expresses the dsRNA adjuvant, but not the antigen. Untreated mice were used as a negative control. The number of animals in each challenge group was 6.

The animals were challenged with a pandemic H1N1 virus (A/CA/07/2009) on week 9 (day 64), using a dose of 8.6x10⁵ TCID₅₀. The A/CA/07/2009 is considered very similar to A/CA/04/09, and the HA proteins share greater than 95% amino acid identity. Animals were measured for weight loss over 14 days post challenge as weight loss is an indicator of the health of the animals.

The results show that DX1-immunized animals were completely protected against death and substantial weight loss (Figures 2A and 2B). P< 0.03 by Fisher's Exact Test when comparing DX1 groups to either untreated or adjuvant control groups for survival. The adjuvant control and untreated mice all lost significant amounts of weight and the majority died as a result of the challenge. Particularly significant are the data showing that a low oral dose of DX1 can still protect 100% of animals, with equivalent weight protection as i.m. immunized animals.

**Table 1**

| Group | Figure 2 Label | Description | N |
|---|---|---|---|
| A | Oral | 2X Oral immunization with 1e8 IU DX1 | 6 |
| B | IM | 2X IM immunization with 1e8 IU DX1 | 6 |
| C | lo dose Oral | 2X Oral immunization with le7 IU DX1 | 6 |
| D | Adj control | 2X Oral immunization 1e8 IU with an adjuvant control vector | 6 |
| E | untreated | untreated | 6 |

### Example 4: Demonstration that oral delivery of DX1 is superior to rAd5 for eliciting antibody responses to H1N1 influenza

A second rAd vector was constructed that expresses the HA from A/CA/04/09, this time without the expressed dsRNA adjuvant. This vector was called DX2. Balb/c mice were dosed by oral gavage on weeks 0 and 4 with 1.0x10⁷ IU DX1 or DX2 (called rAd5 (no adjuvant) in Figure 3). N=6 for this experiment. Antibody titers to A/CA/4/09 HA were measured in the plasma 3 and 7 weeks after administration by anti-HA IgG ELISA (Figure 3). Results show that DX1 induced a superior antibody titer to DX2 demonstrating that the adjuvant improves immune responses to HA following oral delivery.

## Claims

1. A chimeric adenoviral expression vector for the protection against pandemic H1N1 influenza, said vector comprising an expression cassette comprising:
(a) a first promoter operably linked to a nucleic acid encoding a toll-like receptor-3 (TLR-3) agonist, wherein the TLR-3 agonist is a double stranded RNA (dsRNA); and
(b) a second promoter operably linked to a nucleic acid encoding haemagglutinin polypeptide from A/CA/04/2009 H1N1 influenza.

2. The chimeric adenoviral expression vector of claim 1, wherein the nucleic acid encoding the TLR-3 agonist comprises a short hairpin RNA.

3. The chimeric adenoviral expression vector of claim 1, wherein the nucleic acid encoding the TLR-3 agonist comprises a sequence selected from the group consisting of SEQ ID NOs: 1-6.

4. The chimeric adenoviral expression vector according to any one of the preceding claims, wherein the first promoter and the second promoter are the same.

5. The chimeric adenoviral expression vector of claim 4, wherein the first promoter and the second promoter are each a CMV promoter.

6. A pharmaceutical composition comprising the chimeric adenoviral expression vector according to any of the preceding claims and a pharmaceutically acceptable carrier, wherein said pharmaceutical composition is formulated for oral, mucosal, or intranasal administration.

7. An oral delivery pharmaceutical composition comprising the chimeric adenoviral expression vector according to any one of claims 1-5 and a pharmaceutically acceptable carrier.

8. An immunogenically effective amount of a chimeric adenoviral expression vector according to any one of claims 1-5, or a composition according to claims 6 or 7, for use in a method for eliciting an immune response to pandemic H1N1 influenza in a mammal,
wherein the immune response is directed against the haemagglutinin polypeptide, and wherein the route of administration is selected from the group consisting of: oral, intranasal, and mucosal.

9. The expression vector or composition for use in the method of claim 8, wherein the route of administration is oral.

10. The expression vector or composition for use in the method of claim 8 or 9, wherein the immune response is a mucosal immune response.

11. The expression vector or composition for use in the method of any one of claims 8-10, wherein said mammal is a human.

## Patentansprüche

1. Chimärischer adenoviraler Expressionsvektor zum Schutz vor pandemischer H1N1-Influenza, wobei der Vektor eine Expressionskassette umfasst, umfassend:
(a) einen ersten Promotor, der funktionsfähig mit einer Nukleinsäure verknüpft ist, die für einen Agonisten des Tollähnlichen Rezeptors-3 (TLR-3-Agonist) kodiert, wobei der TLR-3-Agonist eine doppelsträngige RNA (dsRNA) ist, und
(b) einen zweiten Promotor, der funktionsfähig mit einer Nukleinsäure verknüpft ist, die für Hämagglutinin-Polypeptid von A/CA/04/2009-H1N1-Influenza kodiert.

2. Chimärischer adenoviraler Expressionsvektor nach Anspruch 1, wobei die Nukleinsäure, die für den TLR-3-Agonisten kodiert, eine Short-Hairpin-RNA umfasst.

3. Chimärischer adenoviraler Expressionsvektor nach Anspruch 1, wobei die Nukleinsäure, die für den TLR-3-Agonisten kodiert, eine Sequenz umfasst, die aus der Gruppe ausgewählt ist bestehend SEQ ID NOs: 1-6.

4. Chimärischer adenoviraler Expressionsvektor nach einem der vorhergehenden Ansprüche, wobei der erste Promotor und der zweite Promotor gleich sind.

5. Chimärischer adenoviraler Expressionsvektor nach Anspruch 4, wobei der erste Promotor und der zweite Promotor jeweils ein CMV-Promotor sind.

6. Pharmazeutische Zusammensetzung, umfassend den chimärischen adenoviralen Expressionsvektor nach einem der vorhergehenden Ansprüche und einen pharmazeutisch unbedenklichen Träger, wobei die pharmazeutische Zusammensetzung zur oralen, mukosalen oder intranasalen Verabreichung formuliert ist.

7. Pharmazeutische Zusammensetzung zur oralen Abgabe, umfassend den chimärischen adenoviralen Expressionsvektor nach einem der Ansprüche 1 bis 5 und einen pharmazeutisch unbedenklichen Träger.

8. Immunogen wirksame Menge eines chimärischen adenoviralen Expressionsvektors nach einem der Ansprüche 1 bis 5 oder einer Zusammensetzung nach einem der Ansprüche 6 oder 7, zur Verwendung in einem Verfahren zum Auslösen einer Immunantwort auf pandemische H1N1-Influenza in einem Säuger,
wobei die Immunantwort gegen Hämagglutinin-Polypeptid gerichtet ist und wobei die Verabreichungsroute ausgewählt ist aus der Gruppe bestehend aus: oral, intranasal und mukosal.

9. Expressionsvektor oder Zusammensetzung zur Verwendung im Verfahren nach Anspruch 8, wobei die Verabreichungsroute oral ist.

10. Expressionsvektor oder Zusammensetzung zur Verwendung im Verfahren nach Anspruch 8 oder 9, wobei die Immunantwort eine mukosale Immunantwort ist.

11. Expressionsvektor oder Zusammensetzung zur Verwendung im Verfahren nach einem der Ansprüche 8 bis 10, wobei der Säuger ein Mensch ist.

## Revendications

1. Vecteur d'expression adénoviral chimère pour la protection contre la grippe H1N1 pandémique, ledit vecteur comprenant une cassette d'expression comprenant :
(a) un premier promoteur fonctionnellement lié à un acide nucléique encodant un agoniste de récepteur de type Toll 3 (TLR-3), dans lequel l'agoniste de TLR-3 est un ARN à double hélice (dsRNA); et
(b) un second promoteur fonctionnellement lié à un acide nucléique encodant le polypeptide d'hémagglutinine de la grippe H1N1 A/CA/04/2009.

2. Vecteur d'expression adénoviral chimère selon la revendication 1, dans lequel l'acide nucléique encodant l'agoniste de TLR-3 comprend un ARN en épingle à cheveux courte.

3. Vecteur d'expression adénoviral chimère selon la revendication 1, dans lequel l'acide nucléique encodant l'agoniste de TLR-3 comprend une séquence sélectionnée parmi le groupe constitué de SEQ ID n° : 1-6.

4. Vecteur d'expression adénoviral chimère selon une quelconque des revendications précédentes, dans lequel le premier promoteur et le second promoteur sont identiques.

5. Vecteur d'expression adénoviral chimère selon la revendication 4, dans lequel le premier promoteur et le second promoteur sont chacun un promoteur de CMV.

6. Composition pharmaceutique comprenant le vecteur d'expression adénoviral chimère selon une quelconque des revendications précédentes et un vecteur de médicament pharmaceutiquement acceptable, dans laquelle ladite composition pharmaceutique est formulée pour l'administration orale, mucosale, ou intranasale.

7. Composition pharmaceutique à administration orale comprenant le vecteur d'expression adénoviral chimère selon une quelconque des revendications 1 à 5 et un vecteur de médicament pharmaceutiquement acceptable.

8. Quantité à efficacité immunogène d'un vecteur d'expression adénoviral chimère selon une quelconque des revendications 1 à 5, ou d'une composition selon les revendications 6 ou 7, pour l'utilisation dans un procédé pour provoquer une réponse immunitaire à la grippe H1N1 pandémique chez un mammifère,
dans laquelle la réponse immunitaire est dirigée contre le polypeptide d'hémagglutinine, et dans laquelle la voie d'administration est sélectionnée parmi le groupe constitué de : orale, intranasale, et mucosale.

9. Vecteur d'expression ou composition pour l'utilisation dans le procédé selon la revendication 8, dans lequel/laquelle la voie d'administration est orale.

10. Vecteur d'expression ou composition pour l'utilisation dans le procédé selon la revendication 8 ou 9, dans lequel/laquelle la réponse immunitaire est une réponse immunitaire mucosale.

11. Vecteur d'expression ou composition pour l'utilisation dans le procédé d'une quelconque des revendications 8 à 10, dans lequel/laquelle ledit mammifère est un humain.
